(19) **Europäisches Patentamt European Patent Office Office européen des brevets**

(11) **EP 1 214 054 B1**

(12) **EUROPEAN PATENT SPECIFICATION**

(45) Date of publication and mention of the grant of the patent:
**31.10.2007 Bulletin 2007/44**

(51) Int Cl.:
*A61K 9/00* (2006.01)      *A61K 9/08* (2006.01)
*A61K 39/145* (2006.01)

(21) Application number: **00967781.6**

(22) Date of filing: **22.09.2000**

(86) International application number:
**PCT/EP2000/009367**

(87) International publication number:
**WO 2001/021151 (29.03.2001 Gazette 2001/13)**

(54) **INTRANASAL INFLUENZA VIRUS VACCINE**

INFLUENZAVIRUS-IMPFSTOFFZUSAMMENSETZUNG ZUR NASALEN ANWENDUNG

VACCIN INTRANASAL CONTRE LES VIRUS GRIPPAUX

(84) Designated Contracting States:
**AT BE CH CY DE DK ES FI FR GB GR IE IT LI LU MC NL PT SE**
Designated Extension States:
**SI**

(30) Priority: **24.09.1999 GB 9922700**
**24.09.1999 GB 9922703**
**06.07.2000 GB 0016686**

(43) Date of publication of application:
**19.06.2002 Bulletin 2002/25**

(73) Proprietor: **GlaxoSmithKline Biologicals s.a.**
**1330 Rixensart (BE)**

(72) Inventors:
• **FRIEDE, Martin**
**SmithKline Beecham Biologicals s.a.**
**B-1330 Rixensart (BE)**
• **HENDERICKX, Veronique**
**SmithKline Beecham Bio. s.a**
**B-1330 Rixensart (BE)**

• **HERMAND, Philippe**
**SmithKline Beecham Bio. s.a.**
**B-1330 Rixensart (BE)**
• **SLAOUI, Moncef Mohamed**
**SmithKline Beecham Bio. s.a**
**B-1330 Rixensart (BE)**
• **THOELEN, Stefan G.J.**
**SmithKline BeechamBio. s.a.**
**B-1330 Rixensart (BE)**

(74) Representative: **Privett, Kathryn Louise et al**
**GlaxoSmithKline,**
**Corporate Intellectual Property (CN9.25.1),**
**980 Great West Road**
**Brentford, Middlesex TW8 9GS (GB)**

(56) References cited:
**WO-A-00/47222          WO-A-94/17827**
**WO-A-96/33738          WO-A-96/36352**
**WO-A-99/52549**

• **DATABASE WPI Section Ch, Week 199815 Derwent Publications Ltd., London, GB; Class B04, AN 1998-167654 XP002158395 & RU 2 086 232 A (UFA VACCINES)**

**Description**

This invention relates to novel non-live split influenza vaccine formulations, methods for preparing them and their use in prophylaxis or therapy. In particular the invention relates to vaccines for administration to the mucosa, more particularly for nasal administration. More particularly the invention relates to the use of non-live-split influenza vaccines which can be administered intranasally in a single dose to achieve a sufficient immune response to meet regulatory requirements.

Influenza virus is one of the most ubiquitous viruses present in the world, affecting both humans and livestock. The economic impact of influenza is significant.

The influenza virus is an RNA enveloped virus with a particle size of about 125 nm in diameter. It consists basically of an internal nucleocapsid or core of ribonucleic acid (RNA) associated with nucleoprotein, surrounded by a viral envelope with a lipid bilayer structure and external glycoproteins. The inner layer of the viral envelope is composed predominantly of matrix proteins and the outer layer mostly of the host-derived lipid material. The surface glycoproteins neuraminidase (NA) and haemagglutinin (HA) appear as spikes, 10 to 12 nm long, at the surface of the particles. It is these surface proteins, particularly the haemagglutinin, that determine the antigenic specificity of the influenza subtypes.

Typical influenza epidemics cause increases in incidence of pneumonia and lower respiratory disease as witnessed by increased rates of hospitalisation or mortality. The elderly or those with underlying chronic diseases are most likely to experience such complications, but young infants also may suffer severe disease. These groups in particular therefore need to be protected.

Currently available influenza vaccines are either inactivated or live attenuated influenza vaccine. Inactivated flu vaccines are composed of three types of antigen preparation: inactivated whole virus, sub-virions where purified virus particles are disrupted with detergents or other reagents to solubilise the lipid envelope (so-called "split" vaccine) or purified HA and NA (subunit vaccine). These inactivated vaccines are given intramuscularly (i.m.).

Influenza vaccines, of all kinds, are usually trivalent vaccines. They generally contain antigens derived from two influenza A virus strains and one influenza B strain. A standard 0.5 ml injectable dose in most cases contains 15 $\mu$g of haemagglutinin antigen component from each strain, as measured by single radial immunodiffusion (SRD) (J.M. Wood et al.: An improved single radial immunodiffusion technique for the assay of influenza haemagglutinin antigen: adaptation for potency determination of inactivated whole virus and subunit vaccines. J. Biol. Stand. 5 (1977) 237-247; J. M. Wood et al., International collaborative study of single radial diffusion and immunoelectrophoresis techniques for the assay of haemagglutinin antigen of influenza virus. J. Biol. Stand. 9 (1981) 317-330).

The influenza virus strains to be incorporated into influenza vaccine each season are determined by the World Health Organisation in collaboration with national health authorities and vaccine manufacturers.

Current efforts to control the morbidity and mortality associated with yearly epidemics of influenza are based on the use of intramuscularly administered inactivated influenza vaccines. The efficacy of such vaccines in preventing respiratory disease and influenza complications ranges from 75% in healthy adults to less than 50% in the elderly.

Influenza viruses, like many pathogens, invade at mucosal surfaces, initially in the upper respiratory tract. Mucosal immunity constitutes the first line of defence for the host and is a major component of the immune response in the nasal passages and in the airways of the lower respiratory tract. Although the presently used injectable influenza vaccines stimulate serum HA-specific IgG in the majority of healthy individuals, a significant rise in HA-specific nasal IgA antibody occurs in only a minority of vaccinated subjects. Improved influenza vaccines with better immunogenicity and clinical efficacy need to target both local and systemic antibody responses.

Experimental intranasal exposure of humans to inactivated influenza vaccines dates back as far as the 1940s (see review in Eyles et al. 2000 BioDrugs 13(1): 35-59). Although there was a resurgence of interest in the use of inactivated virus for IN immunisation in the 1960s and 70s, most attention in the intranasal field has been directed to the live attenuated approach.

Intranasally administered, live attenuated influenza vaccines for example cold-adapted vaccines offer improved mucosal immunity, with promising results particularly in children. However, this approach has failed so far to gain acceptance worldwide.

Thus, most of the commercially available influenza vaccines are either split or subunit injectable vaccines. These vaccines are prepared by disrupting the virus particle, generally with an organic solvent or a detergent, and separating or purifying the viral proteins to varying extents. Split vaccines are prepared by fragmentation of whole influenza virus, either infectious or inactivated, with solubilizing concentrations of organic solvents or detergents and subsequent removal of the solubilizing agent and some or most of the viral lipid material. Split vaccines generally contain contaminating matrix protein and nucleoprotein and sometimes lipid, as well as the membrane envelope proteins. Split vaccines will usually contain most or all of the virus structural proteins although not necessarily in the same proportions as they occur in the whole virus. Subunit vaccines on the other hand consist essentially of highly purified viral surface proteins, haemagglutinin and neuraminidase, which are the surface proteins responsible for eliciting the desired virus neutralising antibodies upon vaccination.

More recently, more highly purified, better characterised split influenza vaccines have been combined with adjuvants in an attempt to improve on the immunogenicity in adults and older people. In spite of significant increases in immune responses in mice, a number of approaches using new generation adjuvants have not proved possible to confirm in man.

Standards are applied internationally to measure the efficacy of influenza vaccines. The European Union official criteria for an effective vaccine against influenza are set out in the table below. Theoretically, to meet the European Union requirements, an influenza vaccine has to meet only one of the criteria in the table, for all strains of influenza included in the vaccine. However in practice, at least two or all three of the criteria will need to be met for all strains, particularly for a new vaccine such as a new intranasal vaccine. Under some circumstances two criteria may be sufficient. For example, it may be acceptable for two of the three criteria to be met by all strains while the third criterion is met by some but not all strains (e.g. two out of three strains). The requirements are different for adult populations (18-60 years) and elderly populations (>60 years).

| | 18 - 60 years | > 60 years |
|---|---|---|
| Seroconversion rate* | >40% | >30% |
| Conversion factor** | >2.5 | >2.0 |
| Protection rate*** | >70% | >60% |

| |
|---|
| * Seroconversion rate is defined as the percentage of vaccinees who have at least a 4-fold increase in serum haemagglutinin inhibition (HI) titres after vaccination, for each vaccine strain. <br> ** Conversion factor is defined as the fold increase in serum HI geometric mean titres (GMTs) after vaccination, for each vaccine strain. <br> *** Protection rate is defined as the percentage of vaccinees with a serum HI titre equal to or greater than 1:40 after vaccination (for each vaccine strain) and is normally accepted as indicating protection. |

For an intranasal flu vaccine to be commercially useful it will not only need to meet those standards, but also in practice it will need to be at least as efficacious as the currently available injectable vaccines. It will also need to be commercially viable in terms of the amount of antigen and the number of administrations required.

Intranasal flu vaccines based on inactivated virus that have been studied over the past few decades have not met these criteria.

Fulk et al. 1969 (J. Immunol. 102, 1102-5) compared intranasal administration (nose drops plus nebulisation) of killed influenza virus with subcutaneous (s.c.) administration in elderly patients. Whereas 56% of the patients receiving s.c. administration exhibited a 4-fold increase in antibody titres (HI), the corresponding increase was observed in only 25% of those receiving an intranasal administration. Two intranasal administrations resulted in a 75% seroconversion.

Gluck et al. 1999 (J. Virol. 73, 7780-6) demonstrated that two consecutive intranasal inoculations administered by a spray containing potent mucosal adjuvants (*E. Coli* Heat Labile Toxin, HLT) were required to induce seroconversion (4-fold increase in humoral antibody response) comparable to an intramuscular administration. A single intranasal administration of 15 $\mu$g HA per strain in the presence of adjuvant, or even two administrations in the absence of adjuvant were incapable of providing equivalent seroconversion. The influenza antigen was in the form of virosomes, reconstituted lipid bilayers produced using phosphatidylcholine and virus surface proteins extracted from egg-derived influenza virus.

The concept of using two or more intranasal administrations in order to attempt to achieve higher levels of seroconversion has also been used by other investigators. Petrescu et al. (1979. Rev. Rom. Med-Virol. 30, 109-115) administered inactivated virus (1000 international units per vaccination dose) intranasally twice over a two week period. Oh et al. (1992 Vaccine 10, 506-11) administered split vaccine in the form of a nasal spray four times at weekly intervals, 15 $\mu$g HA per strain each administration (0.25 ml per nostril on each occasion). Kuno-Sakai et al. (1994. Vaccine 12,1303-1310) administered, twice at an interval of 1 week, aerosol inactivated vaccine threefold the strength of commercially available split influenza.

Documents WO 96/33738 and RU 2 086 232 disclose inactivated influenza compositions that may be suitable for intranasal administration.

Recently Muszkat et al. (2000 Vaccine 18, 1696-9) administered two intranasal immunisations with whole inactivated flu virus (20 $\mu$g of an A strain and a B strain and 40 $\mu$g of another A strain, per dose) to elderly patients and observed lower systemic seroconversion for the intranasal administration than for intramuscular injection of a standard dose of a commercial inactivated split flu vaccine.

Hence the literature spanning 1969 to 2000 shows that although intranasal vaccination with inactivated influenza vaccine has been widely investigated, no group has been able to achieve systemic seroconversion equivalent to intramuscular or subcutaneous injection by administering a single dose of vaccine nasally. Furthermore, in order to achieve an effect with multiple administrations of vaccine over time, the doses of antigen used have been considerably greater than the standard conventional dose of 15 µg HA per strain for each vaccinee. The data in fact point towards a need for multiple administrations, and preferably in the presence of strong immunostimulants such as *E. coli* HLT.

Kimura et al. (1988. Acta Paediatr Jpn. 30, 601-3) demonstrated that administration of two doses of inactivated influenza virus by nebuliser was as effective as a single s.c. administration, but that administration of the two doses by intranasal spray was far less effective. Nebulisation generates a very fine spray which reaches the lungs. Thus there is also an indication in the published clinical trials that a nasal spray may not be effective, and that nebulisation may be better.

The literature further indicates the need for potent adjuvants, more specifically potent immunostimulants, in intranasal vaccines.

For example, Gluck et al. (cited above) demonstrated that the intranasal administration of influenza vaccine in the absence of an immunostimulant is significantly less efficient than in the presence of an immunostimulant. The authors show that even two intranasal administrations of vaccine lacking the immunostimulant are unable to induce the same seroconversion achieved by subcutaneous administration.

Hashigucci et al. 1996 (Vaccine 14, 113-9) demonstrated that two intranasal administrations, four weeks apart, of a split influenza vaccine adjuvanted with a mixture of *E. coli* Heat labile toxin and its B-subunit (LTB) resulted in a seroconversion rate of 50%. In the absence of adjuvant only 31% seroconversion was obtained. Typically s.c. administration results in 75-90% seroconversion.

Thus, the literature clearly indicates that in order to achieve equivalent systemic seroconversion to that obtained with conventional flu vaccines, more than one administration is required, and in addition the vaccine should be adjuvanted with a toxin.

It has now been discovered that non live split influenza virus antigen can be used in a commercially viable intranasal flu vaccine. In particular, a single administration of an intranasal influenza virus vaccine preparation stimulates systemic immunity at a protective level. Furthermore, this meets the international criteria for an effective flu vaccine. More specifically, intranasal administration of a non-live influenza virus antigen preparation can produce a systemic seroconversion (4-fold increase in anti-HA titres) equivalent to that obtained by s.c. administration of the same vaccine. Surprisingly, the influenza antigen can be provided at a significantly lower dose per vaccinee than is indicated in the prior art.

A single nasal administration of a standard dose of inactivated split influenza virus resulting in seroconversion equivalent to that obtained by injection has not previously been reported.

The invention provides for the first time a single administration split influenza vaccine for intranasal delivery. The vaccine meets at least two or all of the EU criteria for influenza vaccines as set out in the table hereinabove for the or all strains of influenza represented in the vaccine, such that the vaccine is approvable in Europe as a commercial one-dose vaccine. More preferably, at least two criteria are met for all strains and the third criterion is met by all strains or at least by all but one of the strains. Most preferably, all strains meet all three of the criteria.

Thus, the invention provides in one aspect the use of a non-live split influenza virus antigen preparation in the manufacture of a vaccine formulation for a one-dose nasal vaccination against influenza wherein the one-dose vaccination achieves an effect as claimed in claim 1. The vaccine may be administered in a mono-dose format or a bi-dose format (generally one sub-dose for each nostril).

The invention provides in another aspect the use of a low dose of non-live split influenza virus antigen material in the manufacture of a mucosal vaccine for immunisation against influenza.

Preferably, the non-live split influenza virus antigen preparation contains at least one surfactant which may be in particular a non-ionic surfactant. Preferably the non-ionic surfactant is at least one surfactant selected from the group consisting of the octyl- or nonylphenoxy polyoxyethanols (for example the commercially available Triton™ series), polyoxyethylene sorbitan esters (Tween™ series) and polyoxyethylene ethers or esters of general formula (I):

$$(I) \qquad HO(CH_2CH_2O)_n\text{-}A\text{-}R$$

wherein n is 1-50, A is a bond or-C(O)-, R is $C_{1-50}$ alkyl or phenyl $C_{1-50}$ alkyl; and combinations of two or more of these.

Preferred surfactants falling within formula (I) are molecules in which n is 4-24, more preferably 6-12, and most preferably 9; the R component is $C_{1-50}$, preferably $C_4\text{-}C_{20}$ alkyl and most preferably $C_{12}$ alkyl.

Octylphenoxy polyoxyethanols and polyoxyethylene sorbitan esters are described in "Surfactant systems" Eds: Attwood and Florence (1983, Chapman and Hall). Octylphenoxy polyoxyethanols (the octoxynols), including t-octylphenoxypolyethoxyethanol (Triton X-100™) are also described in Merck Index Entry 6858 (Page 1162. 12th Edition, Merck & Co. Inc., Whitehouse Station. N.J., USA; ISBN 0911910-12-3). The polyoxyethylene sorbitan esters, including polyoxyethylene sorbitan monooleate (Tween 80™) are described in Merck Index Entry 7742 (Page 1308, 12th Edition, Merck & Co. Inc., Whitehouse Station, N.J., USA; ISBN 0911910-12-3). Both may be manufactured using methods described therein, or purchased from commercial sources such as Sigma Inc.

Particularly preferred non-ionic surfactants include Triton X-45, t-octylphenoxy polyethoxyethanol (Triton X-100), Triton X-102, Triton X-114, Triton X-165, Triton X-205, Triton X-305, Triton N-57, Triton N-101, Triton N-128, Breij 35, polyoxyethylene-9-lauryl ether (laureth 9) and polyoxyethylene-9-stearyl ether (steareth 9). Triton X-100 and laureth 9 are particularly preferred. Also particularly preferred is the polyoxyethylene sorbitan ester, polyoxyethylene sorbitan monooleate (Tween 80™).

Further suitable polyoxyethylene ethers of general formula (I) are selected from the following group: polyoxyethylene-8-stearyl ether, polyoxyethylene-4-lauryl ether, polyoxyethylene-35-lauryl ether, and polyoxyethylene-23-lauryl ether.

Alternative terms or names for polyoxyethylene lauryl ether are disclosed in the CAS registry. The CAS registry number of polyoxyethylene-9 lauryl ether is: 9002-92-0. Polyoxyethylene ethers such as polyoxyethylene lauryl ether are described in the Merck index (12th ed: entry 7717, Merck & Co. Inc., Whitehouse Station, N.J., USA; ISBN 0911910-12-3). Laureth 9 is formed by reacting ethylene oxide with dodecyl alcohol, and has an average of nine ethylene oxide units.

The ratio of the length of the polyoxyethylene section to the length of the alkyl chain in the surfactant (i.e. the ratio of n: alkyl chain length), affects the solubility of this class of surfactant in an aqueous medium. Thus, the surfactants of the present invention may be in solution or may form particulate structures such as micelles or vesicles. As a solution, the surfactants of the present invention are safe, easily sterilisable, simple to administer, and may be manufactured in a simple fashion without the GMP and QC issues associated with the formation of uniform particulate structures. Some polyoxyethylene ethers, such as laureth 9, are capable of forming non-vesicular solutions. However, polyoxyethylene-8 palmitoyl ether ($C_{18}E_8$) is capable of forming vesicles. Accordingly, vesicles of polyoxyethylene-8 palmitoyl ether in combination with at least one additional non-ionic surfactant, can be employed in the formulations of the present invention.

Preferably, the polyoxyethylene ether used in the formulations of the present invention has haemolytic activity. The haemolytic activity of a polyoxyethylene ether may be measured *in vitro,* with reference to the following assay, and is as expressed as the highest concentration of the surfactant which fails to cause lysis of the red blood cells:

1. Fresh blood from guinea pigs is washed with phosphate buffered saline (PBS) 3 times in a desk-top centrifuge. After re-suspension to the original volume the blood is further diluted 10 fold in PBS.
2. 50 $\mu$l of this blood suspension is added to 800 $\mu$l of PBS containing two-fold dilutions of detergent.
3. After 8 hours the haemolysis is assessed visually or by measuring the optical density of the supernatant. The presence of a red supernatant, which absorbs light at 570 nm indicates the presence of haemolysis.
4. The results are expressed as the concentration of the first detergent dilution at which hemolysis no longer occurs.

Within the inherent experimental variability of such a biological assay, the polyoxyethylene ethers, or surfactants of general formula (I), of the present invention preferably have a haemolytic activity, of approximately between 0.5-0.0001 %, more preferably between 0.05-0.0001%, even more preferably between 0.005-0.0001 %, and most preferably between 0.003-0.0004%. Ideally, said polyoxyethylene ethers or esters should have a haemolytic activity similar (i.e. within a ten-fold difference) to that of either polyoxyethylene-9 lauryl ether or polyoxyethylene-8 stearyl ether.

Two or more non-ionic surfactants from the different groups of surfactants described may be present in the vaccine formulation described herein. In particular, a combination of a polyoxyethylene sorbitan ester such as polyoxyethylene sorbitan monooleate (Tween 80™) and an octoxynol such as t-octylphenoxypolyethoxyethanol (Triton) X-100™ is preferred. Another particularly preferred combination of non-ionic surfactants comprises laureth 9 plus a polyoxyethylene sorbitan ester or an octoxynol or both.

Preferably the or each non-ionic surfactant is present in the final vaccine formulation at a concentration of between 0.001 to 20%, more preferably 0.01 to 10%, and most preferably up to about 2% (w/v). Where one or two surfactants are present, these are generally present in the final formulation at a concentration of up to about 2% each, typically at a concentration of up to about 0.6% each. One or more additional surfactants may be present, generally up to a concentration of about 1% each and typically in traces up to about 0.2% or 0.1 % each. Any mixture of surfactants may be present in the vaccine formulations according to the invention.

Non-ionic surfactants such as those discussed above have preferred concentrations in the final vaccine composition as follows: polyoxyethylene sorbitan esters such as Tween 80™: 0.01 to 1%, most preferably about 0.1 % (w/v); octyl- or nonylphenoxy polyoxyethanols such as Triton X-100™ or other detergents in the Triton series: 0.001 to 0.1%, most preferably 0.005 to 0.02 % (w/v); polyoxyethylene ethers of general formula (1) such as laureth 9: 0.1 to 20 %, preferably 0.1 to 10 % and most preferably 0.1 to 1 % or about 0.5% (w/v).

For certain vaccine formulations, other vaccine components may be included in the formulation. As such the formulations of the present invention may also comprise a bile acid or a derivative thereof, in particular in the form of a salt. These include derivatives of cholic acid and salts thereof, in particular sodium salts of cholic acid or cholic acid derivatives. Examples of bile acids and derivatives thereof include cholic acid, deoxycholic acid, chenodeoxycholic acid, lithocholic acid, ursodeoxycholic acid, hyodeoxycholic acid and derivatives such as glyco-. tauro-. amidopropyl-1-propanesulfonic-. amidopropyl-2-hydroxy-1-propanesulfonic derivatives of the aforementioned bile acids. or N.N-bis (3Dgluconoamidopropyl) deoxycholamide. A particularly preferred example is sodium deoxycholate (NaDOC) which may be present in the final vaccine dose.

Preferably, the formulations of the present invention are in the form of an aqueous solution or a suspension of non-vesicular forms. Such formulations are easy to manufacture reproducibly, and also to sterilise (terminal filtration through a 450 or 220 nm pore membrane) and are easy to administer to the nasal mucosa in the form of a spray without degradation of the complex physical structure of the adjuvant.

The non-live flu antigen preparation for use in the invention is a split virus preparation.

In a preferred embodiment, the vaccine formulation comprises a split flu virus preparation in combination with one or more non-ionic surfactants. The one or more non-ionic surfactants may be residual from the process by which the split flu antigen preparation is produced, and/or added to the antigen preparation later. It is believed that the split flu antigen material may be stabilised in the presence of a non-ionic surfactant, though it will be understood that the invention does not depend upon this necessarily being the case.

The invention provides in another aspect the use of a non-live split influenza virus antigen preparation, in the manufacture of a one-dose intranasal influenza vaccine without an added immunostimulant. In the context of this invention, an immunostimulant is a substance which is capable of directly stimulating cells of the immune system, as opposed to only indirectly stimulating e.g. by acting as a carrier for an antigen that itself has a stimulatory effect when in combination with the carrier.

In an alternative aspect of the present invention, the formulation further comprises adjuvants or immunostimulants including Cholera toxin and its B subunit, detoxified lipid A from any source, non-toxic derivatives of lipid A including those described in US 4,912,094, and GB 2,220,211 including non-toxic derivatives of monophosphoryl and diphosphoryl Lipid A such as 3-de-O-acylated monophosphoryl lipid A (3D-MPL) and 3-de-O-acylated diphosphoryl lipid A, saponins such as Quil A (derived from the bark of the South American tree *Quillaja Saponaria Molina*), and fractions thereof, including QS21 and QS17 (US 5,057,540; Kensil, C. R., Crit Rev Ther Drug Carrier Syst, 1996, 12 (1-2):1-55; EP 0 362 279 B1; Kensil et al. (1991. J. Immunology vol 146, 431-437; WO 99/10008) and the oligonucleotide adjuvant system containing an unmethylated CpG dinucleotide (as described in WO 96/02555).

In a preferred embodiment of this aspect of the invention, the formulation comprises a non-toxic lipid A derivative selected from 3D-MPL and non-toxic derivatives of diphosphoryl lipid A, particularly 3D-MPL. More preferably, the formulation comprises 3D-MPL together with a polyoxythylene ether or ester of general formula (I) as defined hereinabove, in particular laureth 9.

Thus, the invention further provides a vaccine comprising a combination of 3D-MPL and laureth 9. and an influenza virus antigen preparation, particularly a split antigen preparation. This vaccine is particularly, though not exclusively, suitable for mucosal administration including intranasal administration as described herein.

Additional components that are preferably present in the formulation according to this aspect of the invention include further non-ionic detergents such as the octoxynols and polyoxyethylene esters as described herein, particularly t-octylphenoxy polyethoxyethanol (Triton X-100) and polyoxyethylene sorbitan monooleate (Tween 80): and bile salts or cholic acid derivatives as described herein, in particular sodium deoxycholate or taurodeoxycholate. Thus, a particularly preferred formulation comprises 3D-MPL. laureth 9. Triton X- 100. Tween 80 and sodium deoxycholate. which may be combined with a split influenza virus antigen preparation to provide a vaccine suitable for mucosal or intranasal application.

The invention also provides a method for manufacturing a vaccine comprising admixing 3D-MPL, laureth 9 and a split influenza virus antigen preparation such as a split antigen preparation employed in a conventional intramuscular influenza vaccine.

In a further aspect, the invention provides a pharmaceutical kit comprising an intranasal spray device and a one-dose non-live split influenza virus vaccine which generates an immune response that achieves at least two out of the criteria as set forth in claim 1 for the or all influenza strains present in the vaccine. Preferably the device is a bi-dose delivery device for two sub-doses of vaccine.

The low dose of haemagglutinin according to the invention is preferably a haemagglutinin dose comparable to the dose in the current commercial flu vaccines. Thus the preferred low dose is preferably not more than about 30 μg, more preferably not more than about 15 μg of haemagglutinin per influenza strain. This equates to normally somewhere between 0.1 and 2 μg /kg bodyweight. Preferably but not necessarily the low dose vaccines of the invention are administered as a one-dose vaccine e.g. in two sub-doses, one for each nostril.

Advantageously, a vaccine dose according to the invention is provided in a smaller volume than the conventional injected split flu vaccines, which are generally 0.5 or 1 ml per dose. The low volume doses according to the invention are preferably below 500 μl, more preferably below 300 μl and most preferably not more than about 200 μl or less per

dose. When two sub-doses are given, the preferred volume per sub-dose is half of the total dose volumes mentioned above.

Thus, a preferred vaccine dose according to the invention is a dose with a low antigen dose in a low volume, e.g. about 15 $\mu$g or about 7.5 $\mu$g HA (per strain) in a volume of about 200 $\mu$l.

The invention also discloses a method for the prophylaxis of influenza infection or disease in a subject which method comprises administering to the subject a one-dose non-live influenza vaccine via a mucosal surface.

The invention further discloses a method for prophylaxis of influenza infection or disease in a subject which method comprises administering to the subject a low dose of a non-live influenza virus vaccine via a mucosal surface.

Preferably the vaccine is administered intranasally.

Most preferably, the vaccine is administered locally to the nasopharyngeal area, preferably without being inhaled into the lungs. It is desirable to use an intranasal delivery device which delivers the vaccine formulation to the nasopharyngeal area, without or substantially without it entering the lungs.

Preferred devices for intranasal administration of the vaccines according to the invention are spray devices. Suitable commercially available nasal spray devices include Accuspray™ (Becton Dickinson). Nebulisers produce a very fine spray which can be easily inhaled into the lungs and therefore does not efficiently reach the nasal mucosa. Nebulisers are therefore not preferred.

Preferred spray devices for intranasal use are devices for which the performance of the device is not dependent upon the pressure applied by the user. These devices are known as pressure threshold devices. Liquid is released from the nozzle only when a threshold pressure is applied. These devices make it easier to achieve a spray with a regular droplet size. Pressure threshold devices suitable for use with the present invention are known in the art and are described for example in WO 91/13281 and EP 311 863 B and EP 516 636. Such devices are commercially available from Pfeiffer GmbH and are also described in Bommer, R. Pharmaceutical Technology Europe, Sept 1999.

Preferred intranasal devices produce droplets (measured using water as the liquid) in the range 1 to 200$\mu$m, preferably 10 to 120$\mu$m. Below 10$\mu$m there is a risk of inhalation, therefore it is desirable to have no more than about 5% of droplets below 10$\mu$m. Droplets above 120$\mu$m do not spread as well as smaller droplets, so it is desirable to have no more than about 5% of droplets exceeding 120$\mu$m.

Bi-dose delivery is a further preferred feature of an intranasal delivery system for use with the vaccines according to the invention. Bi-dose devices contain two sub-doses of a single vaccine dose, one sub-dose for administration to each nostril. Generally, the two sub-doses are present in a single chamber and the construction of the device allows the efficient delivery of a single sub-dose at a time. Alternatively, a monodose device may be used for administering the vaccines according to the invention.

The invention provides in a further aspect a pharmaceutical kit comprising an intranasal administration device as described herein containing a vaccine formulation according to the invention.

The invention is not necessarily limited to spray delivery of liquid formulations. Vaccines according to the invention may be administered in other forms e.g. as a powder.

The influenza vaccine according to the invention is preferably a multivalent influenza vaccine comprising two or more strains of influenza. Most preferably it is a trivalent vaccine comprising three strains. Conventional influenza vaccines comprise three strains of influenza, two A strains and one B strain. However, monovalent vaccines, which may be useful for example in a pandemic situation, are not excluded from the invention. A monovalent, pandemic flu vaccine will most likely contain influenza antigen from a single A strain.

The non-live split influenza virus preparations may be derived from the conventional embryonated egg method, or they may be derived from any of the new generation methods using tissue culture to grow the virus. Suitable cell substrates for growing the virus include for example dog kidney cells such as MDCK or cells from a clone of MDCK, MDCK-like cells, monkey kidney cells such as AGMK cells including Vero cells, or any other mammalian cell type suitable for the production of influenza virus for vaccine purposes. Suitable cell substrates also include human cells e.g. MRC-5 cells. Suitable cell substrates are not limited to cell lines; for example primary cells such as chicken embryo fibroblasts are also included.

The influenza virus antigen preparation may be produced by any of a number of commercially applicable processes, for example the split flu process described in patent no. DD 300 833 and DD 211 444. Traditionally split flu was produced using a solvent/detergent treatment, such as tri-*n*-butyl phosphate, or diethylether in combination with Tween™ (known as "Tween-ether" splitting) and this process is still used in some production facilities. Other splitting agents now employed include detergents or proteolytic enzymes or bile salts, for example sodium deoxycholate as described in patent no. DD 155 875. Detergents that can be used as splitting agents include cationic detergents e.g. cetyl trimethyl ammonium bromide (CTAB), other ionic detergents e.g. laurylsulfate, taurodeoxycholate, or non-ionic detergents such as the ones described above including Triton X-100 (for example in a process described in Lina et al, 2000, Biologicals 28, 95-103) and Triton N-101, or combinations of any two or more detergents.

Further suitable splitting agents which can be used to produce split flu virus preparations include:

1. Bile acids and derivatives thereof including: cholic acid, deoxycholic acid, chenodeoxy colic acid, lithocholic acid ursodeoxycholic acid, hyodeoxycholic acid and derivatives like glyco-, tauro-, amidopropyl-1-propanesulfonic-, amidopropyl-2-hydroxy-1-propanesulfonic derivatives of the aforementioned bile acids, or N,N-bis (3DGluconoamidopropyl) deoxycholamide. A particular example is sodium deoxycholate (NaDOC) which may be present in trace amounts in the final vaccine dose.

2. Alkylglycosides or alkylthioglycosides, where the alkyl chain is between C6 - C18 typical between C8 and C14, sugar moiety is any pentose or hexose or combinations thereof with different linkages, like 1-> 6, 1->5, 1->4, 1->3, 1-2. The alkyl chain can be saturated unsaturated and/or branched.

3. Derivatives of 2 above, where one or more hydroxyl groups, preferably the 6 hydroxyl group is/are modified, like esters, ethoxylates, sulphates, ethers, carbonates, sulphosuccinates, isethionates, ethercarboxylates, quarternary ammonium compounds.

4. Acyl sugars, where the acyl chain is between C6 and C18, typical between C8 and C12, sugar moiety is any pentose or hexose or combinations thereof with different linkages, like 1-> 6. 1->5, 1->4, 1->3, 1-2. The acyl chain can be saturated or unsaturated and/or branched, cyclic or non-cyclic, with or without one or more heteroatoms e.g. N, S, P or O.

5. Sulphobetaines of the structure R-N,N-(R1,R2)-3-amino-1-propanesulfonate, where R is any alkyl chain or arylalkyl chain between C6 and C18, typical between C8 and C16. The alkyl chain R can be saturated, unsaturated and/or branched. R1 and R2 are preferably alkyl chains between C1 and C4, typically C1, or R1, R2 can form a heterocyclic ring together with the nitrogen.

6. Betains of the structure R-N,N-(R1.R2)-glycine, where R is any alkyl chain between C6 and C18, typical between C8 and C16. The alkyl chain can be saturated unsaturated and/or branched. R1 and R2 are preferably alkyl chains between C1 and C4, typically C1. or R1 and R2 can form a heterocyclic ring together with the nitrogen.

7. N,N-dialkyl-glucamides, of the Structure R-(N-R1)-glucamide, where R is any alkylchain between C6 and C18, typical between C8 and C12. The alkyl chain can be saturated unsaturated and/or branched or cyclic. R1 and R2 are alkyl chains between C1 and C6, typically C1. The sugar moiety might be modified with pentoses or hexoses.

8. Quarternary ammonium compounds of the structure R. -$N^+$ (-R1, -R2, -R3), where R is any alkylchain between C6 and C20, typically C20. The alkyl chain can be saturated unsaturated and/or branched. R1, R2 and R3 are preferably alkyl chains between C1 and C4, typically C1, or R1, R2 can form a heterocyclic ring together with the nitrogen. A particular example is cetyl trimethyl ammonium bromide (CTAB).

The preparation process for a split vaccine will include a number of different filtration and/or other separation steps such as ultracentrifugation, ultrafiltration, zonal centrifugation and chromatography (e.g. ion exchange) steps in a variety of combinations, and optionally an inactivation step eg with formaldehyde or β-propiolactone or U.V. which may be carried out before or after splitting. The splitting process may be carried out as a batch, continuous or semi-continuous process.

Preferably, a bile salt such as sodium deoxycholate is present in trace amounts in a split vaccine formulation according to the invention, preferably at a concentration not greater than 0.05%, or not greater than about 0.01 %, more preferably at about 0.0045% (w/v).

Preferred split flu vaccine antigen preparations according to the invention comprise a residual amount of Tween 80 and/or Triton X-100 remaining from the production process, although these may be added or their concentrations adjusted after preparation of the split antigen. Preferably both Tween 80 and Triton X-100 are present. The preferred ranges for the final concentrations of these non-ionic surfactants in the vaccine dose are:

Tween 80: 0.01 to 1%, more preferably about 0.1 % (v/v)
Triton X-100: 0.001 to 0.1 (% w/v), more preferably 0.005 to 0.02% (w/v).

The presence of the combination of these two surfactants, in low concentrations, was found to promote the stability of the antigen in solution. It is possible that this enhanced stability rendered the antigen more immunogenic nasally than previous formulations have been. Such an enhancement could arise from a prevalence of small antigen aggregates or the enhancement of the native conformation of the antigen. It will be appreciated that the invention does not depend upon this theoretical explanation being correct.

There is also evidence to show that in the case of a split influenza virus preparation, the presence of fragments of whole influenza virus associated with the viral proteins or containing the viral proteins, in particular HA, may preserve the presentation of the antigen and may contribute to inducing a strong immune response. Thus, split influenza virus is the antigen of choice for use in the various aspects of the present invention.

In a particular embodiment, the preferred split virus preparation also contains laureth 9, preferably in the range 0.1 to 20%, more preferably 0.1 to 10% and most preferably 0.1 to 1% (w/v).

The vaccines according to the invention generally contain not more than 25% (w/v) of detergent or surfactant, preferably less than 15% and most preferably not more than about 2%.

The invention provides in another aspect a method of manufacturing a one-dose influenza vaccine for nasal application which method comprises:

(i) providing a split influenza virus preparation produced essentially as for a conventional injected (e.g.intramuscular) influenza vaccine and comprising at least one non-ionic surfactant;
(ii) optionally adjusting the concentration of the haemagglutinin and/or the concentration of non-ionic surfactant in the preparation;
(iii) filling an intranasal delivery device with a vaccine dose from the split influenza virus preparation, said dose being a suitable volume for intranasal administration, optionally in a bi-dose format, and which one-dose application generates an immune response that achieves at least two of the criteria as set forth in claim 1 for the or all influenza strains present in the vaccine.

A further optional step in the method according to this aspect of the invention includes the addition of an absorption-enhancing surfactant such as laureth 9, and/or the addition of an adjuvant such as a non-toxic lipid A derivative, especially 3D-MPL.

Processes for producing conventional injected inactivated flu vaccines are well known and described in the literature. Such processes may be modified for producing a one-dose mucosal vaccine for use in the present invention, for example by the inclusion of a concentration step prior to final sterile filtration of the vaccine, since intranasal vaccines advantageously employ a smaller volume of vaccine formulation than injected vaccines. Or the process may be modified by the inclusion of a step for adjusting the concentration of other components e.g. non-ionic surfactants to a suitable % (w/v) for an intranasal vaccine according to the invention. However, the active ingredient of the vaccine, i.e. the influenza antigen can be essentially the same for the conventional intramuscular vaccine and the one-dose intranasal vaccines according to the invention.

The invention will now be further described in the following; non-limiting examples.

## Example 1 - Preparation of split influenza vaccine

Monovalent split vaccine was prepared according to the following procedure.

### Preparation of virus inoculum

On the day of inoculation of embryonated eggs a fresh inoculum is prepared by mixing the working seed lot with a phosphate buffered saline containing gentamycin sulphate at 0.5 mg/ml and hydrocortisone at 25 $\mu$g/ml. (virus strain-dependent). The virus inoculum is kept at 2-8°C.

### Inoculation of embryonated eggs

Nine to eleven day old embryonated eggs are used for virus replication. Shells are decontaminated. The eggs are inoculated with 0.2 ml of the virus inoculum. The inoculated eggs are incubated at the appropriate temperature (virus strain-dependent) for 48 to 96 hours. At the end of the incubation period, the embryos are killed by cooling and the eggs are stored for 12-60 hours at 2-8°C.

### Harvest

The allantoic fluid from the chilled embryonated eggs is harvested. Usually. 8 to 10 ml of crude allantoic fluid is collected per egg. To the crude monovalent virus bulk 0.100 mg/ml thiomersal is optionally added.

**Concentration and purification of whole virus from allantoic fluid**

### 1. Clarification

The harvested allantoic fluid is clarified by moderate speed centrifugation (range: 4000 - 14000 g).

### 2. Adsorption step

To obtain a CaHPO$_4$ gel in the clarified virus pool, 0.5 mol/L Na$_2$HPO$_4$ and 0.5mol/L CaCl$_2$ solutions are added to reach a final concentration of CaHPO$_4$ of 1.5 g to 3.5 g CaHPO$_4$/litre depending on the virus strain.
After sedimentation for at last 8 hours, the supernatant is removed and the sediment containing the influenza virus is resolubilised by addition of a 0.26 mol/L EDTA-Na, solution, dependent on the amount of CaHPO$_4$ used.

### 3. Filtration

The resuspended sediment is filtered on a 6$\mu$m filter membrane.

### 4. Sucrose gradient centrifugation

The influenza virus is concentrated by isopycnic centrifugation in a linear sucrose gradient (0.55 % (w/v)) containing 100 $\mu$g/ml Thiomersal. The flow rate is 8 - 15 litres/hour.
At the end of the centrifugation, the content of the rotor is recovered by four different fractions (the sucrose is measured in a refractometer):

- fraction 1 55-52% sucrose
- fraction 2 approximately 52-38% sucrose
- fraction 3 38-20% sucrose*
- fraction 4 20- 0% sucrose

* virus strain-dependent: fraction 3 can be reduced to 15% sucrose.
For further vaccine preparation, only fractions 2 and 3 are used.
Fraction 3 is washed by diafiltration with phosphate buffer in order to reduce the sucrose content to approximately below 6%. The influenza virus present in this diluted fraction is pelleted to remove soluble contaminants.
The pellet is resuspended and thoroughly mixed to obtain a homogeneous suspension. Fraction 2 and the resuspended pellet of fraction 3 are pooled and phosphate buffer is added to obtain a volume of approximately 40 litres. This product is the monovalent whole virus concentrate.

### 5. Sucrose gradient centrifugation with sodium deoxycholate

The monovalent whole influenza virus concentrate is applied to a ENI-Mark II ultracentrifuge. The K3 rotor contains a linear sucrose gradient (0.55 % (w/v)) where a sodium deoxycholate gradient is additionally overlayed. Tween 80 is present during splitting up to 0.1 % (w/v). The maximal sodium deoxycholate concentration is 0.7-1.5 % (w/v) and is strain dependent. The flow rate is 8 - 15 litres/hour.
At the end of the centrifugation, the content of the rotor is recovered by three different fractions (the sucrose is measured in a refractometer) Fraction 2 is used for further processing. Sucrose content for fraction limits (47-18%) varies according to strains and is fixed after evaluation:

### 6. Sterile filtration

The split virus fraction is filtered on filter membranes ending with a 0.2 $\mu$m membrane. Phosphate buffer containing 0.025 % (w/v) Tween 80 is used for dilution. The final volume of the filtered fraction 2 is 5 times the original fraction volume.

### 7. Inactivation

The filtered monovalent material is incubated at 22 $\pm$ 2°C for at most 84 hours (dependent on the virus strains, this incubation can be shortened). Phosphate buffer containing 0.025% Tween 80 is then added in order to reduce the total protein content down to max. 250 $\mu$g/ml. Formaldehyde is added to a final concentration of 50 $\mu$g/ml and the

inactivation takes place at 20°C $\pm$ 2°C for at least 72 hours.

### 8. Ultrafiltration

The inactivated split virus material is concentrated at least 2 fold in a ultrafiltration unit, equipped with cellulose acetate membranes with 20 kDa MWCO. The Material is subsequently washed with phosphate buffer containing 0.025 % (w/v) Tween 80 and following with phosphate buffered saline containing 0.01 % (w/v) Tween.

### 9. Final sterile filtration

The material after ultrafiltration is filtered on filter membranes ending with a 0.2 $\mu$m membrane. The final concentration of Haemagglutinin, measured by SRD (method recommended by WHO) should exceed 450 $\mu$g/ml.

### 10. Storage

The monovalent final bulk is stored at 2 - 8°C for a maximum of 18 months.

### Purity

Purity was determined by O.D. scanning of Coomassie-stained polyacrylamide gels. Peaks were determined manually. Sample results are given in Table 1:

**Table I**

| Viral Proteins (HA, NP, M) % | | | | | Other viral and host-cell derived proteins % |
|---|---|---|---|---|---|
| **H3N2** | **HA dimer** | **HA1 + 2** | **NP** | **M** | |
| A/Syd/5/97 | 10.34 | 22.34 | 25.16 | 37.33 | 4.83 |
| A/Nan933/95 | 8.17 | 15.8 | 40.09 | 30.62 | 5.32 |
| **B** | | | | | |
| B/Har/7/94 | 5.71[2] | 24.07 | 15.64 | 50 | 4.58 |
| B/Yam/166/98 | 0.68 | 27.62 | 21.48 | 46.02 | 4.2 |
| **H1N1** | | | | | |
| A/Tex/36/91 | | 33.42 | 24.46 | 34.33 | 7.79 |
| A/Bei/262/95 | | 32.73 | 35.72 | 27.06 | 4.49 |
| **H2N2** | | | | | |
| A/sing/1/57 | 2.8 | 39.7 | 21.78 | 32.12 | 3.6 |
| [1] = 100 % minus all non-identified peaks | | | | | |

### Example 2 - Preparation of vaccine doses from bulk vaccine

Final vaccine is prepared by formulating a trivalent vaccine from the monovalent bulk with the detergent concentrations adjusted as required.

Water for injection, PBS pH 7.4 10x concentrated, Tween 80 and Triton X-100 are mixed to obtain the required final concentrations (PBS 1x concentrated, Tween 80 0.15% and Triton X-100 0.02%) . The three following inactivated split virions are added with 10 minutes stirring in between:

30$\mu$g HA A/Beijing/262/95 (H1N1)
30$\mu$g HA A/Sydney/5/97 (H3N2)
30$\mu$g HA B/Harbin/7/94

After 15 minutes stirring pH is adjusted to 7.2+/-0.2.
The dose volume is 200$\mu$l.
In the vaccine formulated with laureth 9, the laureth 9 is added prior to pH adjustment to obtain a final concentration of 0.5% (w/v).

**Example 3 - Methods used to measure antibody responses**

**1. Detection of specific anti-Flu and total IgA in human nasal secretions by ELISA**

*Collection method for human nasal secretions*

Two wicks are applied against the inferior turbinate (one in each nostril) of the volunteer. Wicks are left in the nose for 1 minute before being placed in 2 ml of NaCl 0.9%. BSA 1% and sodium azide 0.1 % (preservative buffer). All the samples are left for a 2 hours period on ice. The wicks are then pressed to recover the antibodies. Following centrifugation (10', 2000g. 4°C) the fluids of all samples are collected, aliquoted and frozen at -20°C until the date of test. The pellets are suspended in 400$\mu$l of physiological water and microscopically observed for blood cells contamination.

After collection using nasal wicks and treatment of human nasal secretions, the detection of total and specific anti-FLU IgA is realized with two different ELISAs:

*Capture ELISA for detection of total IgA*

Total IgA are captured with anti-human IgA polyclonal affinity purified Ig immobilized on microtiter plates and subsequently detected using a different polyclonal anti-human IgA affinity purified Ig coupled to peroxidase.
A purified human sIgA is used as a standard to allow the quantification of sIgA in the collected nasal secretions.
3 references of purified human sIgA are used as low, medium and high references in this assay.

*Direct ELISA for detection of specific anti-FLU IgA*

Three different ELISAs are performed, one on each FLU strain present in the vaccine formulation.
Specific anti-FLU IgA are captured with split inactivated FLU antigens coated on microtiter plates and subsequently detected using the same different polyclonal anti-human IgA affinity purified Ig coupled to peroxidase as the one used for the total IgA ELISA.

**Reagents**

*Biological reagents*

- Goat anti-Human IgA affinity purified Ig.(Sigma 1-0884)

- Purified Human secretory IgA (ICN-Cappel 55905) (Standard for total IgA quantification)

- Human secretory IgA (Colostrum) (Biogenesis 5111-5504) (reference Bio for total IgA), diluted to obtain low, medium and high references

- Purified Human IgA (Sigma I-1010) (reference Sig for total IgA)

- Negative reference for specific anti-FLU ELISA (pool of nasal secretions with undetectable responses against the 3 strains; cut-off = 0.6 OD$_{450nm}$)

- Positive low reference for specific anti- FLU ELISA (pool of nasal secretions with low detectable responses against the 3 strains)

- Positive medium reference for specific anti- FLU ELISA (pool of nasal secretions with medium detectable responses against the 3 strains; cut-off = 0.6 OD)

- Goat anti-Human IgA serum affinity purified HRP conjugated (ICN 674221)

- Split inactivated egg derived antigen A/Beijing/262/95 H1N1

- Split inactivated egg derived antigen A/Sydney/5/97 H3N2

- Split inactivated egg derived antigen B/Harbin/7/94

*Reagents preparation*

- Saturation buffer (PBS. Tween 20 0.1%, BSA 1%, NCS 4%)

- NaCl T20 (NaCl 9g/l, Tween 20 0.05%)

**Method**

*Total human IgA detection*

- Add 100 $\mu$l/well of goat polyclonal anti human IgA at 1 $\mu$g/ml in DPBS and incubate overnight at 4°C.

- Add 200 $\mu$l/well of saturation buffer and incubate for 1 hour at 37°C.

- Add in the first row: 100 $\mu$l/well of two-fold dilutions of the standard IgA in saturation buffer starting from 250 ng/ml down to 0.12 ng/ml.

- Add in the other rows: 100 $\mu$l/well of two-fold dilutions of the samples (nasal fluids) in saturation buffer starting from 1/100 down to 1/102400, add 100$\mu$l of saturation buffer in the column 12 and incubate for 2 hours at 22°C.

- Wash the plates four times in NaCl T20.

- Add 100 $\mu$l/well of goat peroxidase-conjugated anti human IgA diluted in saturation buffer at 1/10000 and incubate for 1 1/2 hour at 22°C.

- Wash the plates four times in NaCl T20.

- Add 100 $\mu$l/well of TMB (tetramethylbenzidine) and incubate at room temperature for 10 min. in the dark.

- Stop the reaction by adding 100$\mu$l/well of $H_2SO_4$ 0.4N.

- Measure the absorbance (OD) of each plate using a spectrophotometer at 450 nm with a reference at 630 nm.

*Specific anti-FLU IgA detection*

- Add 100 $\mu$l/well of each strain of FLU virus at 1 $\mu$g/ml in DPBS and incubate overnight at 4°C.

- Add 200 $\mu$l/well of saturation buffer and incubate for 1 hour at 37°C.

- Add 100 $\mu$l/well of two-fold dilutions of the samples in saturation buffer starting from 1/5 down to 1/640 and incubate the plates for 2 hours at 22°C.

- Wash the plates four times in NaCl T20.

- Add 100 $\mu$l/well of goat peroxidase-conjugated anti human IgA diluted in saturation buffer at 1/10000 and incubate for 1/2 hour at 22°C.

- Wash the plates four times in NaCl T20.

- Add 100 $\mu$l/well of TMB (tetramethylbenzidine) and incubate at room temperature for 10 min. in the dark.

- Stop the reaction by adding 100$\mu$l/well of $H_2SO_4$ 0.4N.

- Measure the absorbance (OD) of each plate using a spectrophotometer at 450 nm with a reference at 630 nm.

**Results - expression and calculations**

*Total IgA expression*

The results are expressed as $\mu$g of total IgA in 1 ml of nasal fluids, using a Softmaxpro program.

*Specific anti-Flu IgA expression*

The results are expressed as end-point unit titer, which are calculated as the inverse of the last dilution which gives an $OD_{450nm}$ above the cut off ($OD_{450nm}$ = 0.6).
The cut off value is defined as the highest optical density of the negative reference (see validation protocol) at a dilution of 1/5. The limit of detection corresponding to the end-point unit titer at the cut off can thus be calculated as being 5 end-point units. Samples with a titer $\leq$ 5 end-point unit will be considered as negative and samples with a titer > 5 end-point unit will be considered as positive.
The final results of a sample are expressed as follows:
Normalization of the specific response by calculating the ratio between the specific response and the total IgA concentration: end-point unit/$\mu$g total IgA (most commonly used calculation method in the literature).

**2. Haemagglutination Inhibition (HAI) activity of Flu-specific serum Abs**

Sera (50 $\mu$l) are treated with 200 $\mu$l RDE (receptor destroying enzyme) for 16 hours at 37°C. The reaction is stopped with 150 $\mu$l 2.5% Na citrate and the sera are inactivated at 56°C for 30 min. A dilution 1:10 is prepared by adding 100 $\mu$l PBS. Then, a 2-fold dilution series is prepared in 96 well plates (V-bottom) by diluting 25 $\mu$l serum (1:10) with 25 $\mu$l PBS. 25 $\mu$l of the reference antigens are added to each well at a concentration of 4 hemagglutinating units per 25 $\mu$l. Antigen and antiserum dilution are mixed using a microtiter plate shaker and incubated for 60 minutes at room temperature. 50 $\mu$l chicken red blood cells (RBC) (0.5%) are then added and the RBCs are allowed to sediment for 1 hour at RT. The HAI titre corresponds to the inverse of the last serum dilution that completely inhibits the virus-induced hemagglutination.

**Example 4 - A comparison of the immunogenicity of an intranasal split influenza vaccine with that of a licensed conventional parenteral vaccine (Fluarix™) in healthy adult subjects.**

*Formulations used in the study*

Two formulations (A,B) of egg-derived split influenza antigens were evaluated. A is an intranasal formulation and B is the Fluarix™/$\alpha$-Rix® given intramuscularly. The formulations contain three inactivated split virion antigens prepared from the WHO recommended strains of the 1998/1999 season.
The device used for administration of the vaccines was the Accuspray™ intranasal syringe from Becton Dickinson. The device works on a similar basis to a conventional syringe, but has a special tip containing spiral channels which result in the production of a spray when even pressure is exerted on the plunger. The device was filled with 200$\mu$l of vaccine formulation, and 100$\mu$l of the A formulation was sprayed in each nostril.

Composition of the formulations.

The intranasal formulation (A) contained the following inactivated split virions:

1. 30$\mu$g HA A/beijing/262/95 (H1N1)
2. 30$\mu$g HA A/Sydney/5/97 (H3N2)
3. 30$\mu$g HA of B/Harbin/7/94

and phosphate buffered saline pH 7.4$\pm$ 0.1, Tween 80 0.1%, Triton X-100 0.015%, Na deoxycholate 0.0045% and thiomersal below 35$\mu$g/ml.
The volume of one dose was 200$\mu$l (100$\mu$l sub-doses for each nostril).

The comparator Fluarix™/α-Rix® is the SmithKline Beecham Biologicals' commercial inactivated trivalent split influenza vaccine. The dose of 500μl was administered intramuscularly.

This dose contains:

15μg HA of the three strains mentioned above, Tween 80 between 500 and 1000 μg per ml (0.05%-0.1 %). Triton X-100 between 50 and 170μg/ml (0.005%-0.017%), sodium deoxycholate maximum 100μg/ml, thiomersal 100μg/ml and phosphate buffered saline pH between 6.8 and 7.5.

## Immunogenicity Study

An open, controlled and randomised study evaluated the immunogenicity of an intranasal split influenza vaccine formulated with Tween 80 & Triton X-100 compared to the conventional parenteral vaccine (i.e. Fluarix™). Twenty healthy adult subjects (aged 18-40 years) received one dose of Fluarix™ and ten subjects received one dose of the intranasal influenza vaccine. The intranasal formulation (200μl) contained the following inactivated virions: 30μg of haemagglutinin A/Beijing/262/95 (H1N1), 30μg of haemagglutinin A/Sydney/5/97 (H3N2). 30μg of haemagglutinin B/Harbin/7/94 with phosphate buffered saline (pH 7.4 ± 0.1). Tween 80 (0.1%), Triton X-100 (0.015%), sodium deoxycholate (0.0045%) and thiomersal (<35μg/ml).

There was an eight-day follow-up period for solicited local and general symptoms and both vaccines were well-tolerated regarding safety and reactogenicity. No serious adverse events related to vaccination were reported.

The immunogenicity of the vaccines was examined by assessing the serum haemagglutination inhibition (HI) titres to determine the seroconversion rate (defined as the percentage of vaccinees who have at least a 4-fold increase in serum HI titres on day 21 compared to day 0. for each vaccine strain), conversion factor (defined as the fold increase in serum HI Geometric Mean Titres (GMTs) on day 21 compared to day 0, for each vaccine strain) and seroprotection rate (defined as the percentage of vaccinees with a serum HI titre ≥40 after vaccination (for each vaccine strain) that is accepted as indicating protection). In addition, the mucosal IgA antibody response was assessed by Enzyme Linked Immunosorbent Assay (ELISA).

HI seropositivity, serconversion and seroprotection rates twenty-one days after one dose of Fluarix™ or the intranasal formulation can be seen in Table 2. Conversion factor can be seen from Table 2a.

**Table 2:**

| HI seropositivity, serconversion and seroprotection rates at 21 days post dose 1 | | | | | | | | | |
|---|---|---|---|---|---|---|---|---|---|
| *Strain* | **Group** | *Timing* | **N** | **Seropositivity** | | **Seroprotection** | | **Seroconversion** | |
| | | | | n | % | n | % | n | % |
| **A/Beijing** | Intranasal vaccine plus Tween 80 & Titron X 100 | Day 0 | 20 | 4 | 20.0 | 0 | 0.0 | | |
| | | Day 21 | 20 | 17 | 85.0 | 15 | 75.0 | 15 | 75.0 |
| | Fluarix™ | Day 0 | 19 | 4 | 21.1 | 3 | 15.8 | | |
| | | Day 21 | 19 | 19 | 100.0 | 18 | 94.7 | 19 | 100.0 |
| **A/Sydney** | Intranasal vaccine plus Tween 80 & Titron X100 | Day 0 | 20 | 13 | 65.0 | 3 | 15.0 | | |
| | | Day 21 | 20 | 20 | 100.0 | 19 | 95.0 | 15 | 75.0 |
| | Fluarix™ | Day 0 | 19 | 14 | 73.7 | 1 | 5.3 | | |
| | | Day 21 | 19 | 19 | 100.0 | 18 | 94.7 | 16 | 84.2 |

(continued)

| | | | | Seropositivity | | Seroprotection | | Seroconversion | |
|---|---|---|---|---|---|---|---|---|---|
| *Strain* | **Group** | *Timing* | **N** | n | % | n | % | n | % |
| B/Harbin | Intranasal vaccine plus Tween 80 & Titron X 100 | Day 0 | 20 | 10 | 50.0 | 7 | 35.0 | | |
| | | Day 21 | 20 | 20 | 100.0 | 18 | 90.0 | 14 | 70.0 |
| | Fluarix™ | Day 0 | 19 | 17 | 89.5 | 11 | 57.9 | | |
| | | Day 21 | 19 | 19 | 100.0 | 19 9 | 100.0 | 15 | 78.9 |

*HI seropositivity, serconversion and seroprotection rates at 21 days post dose 1*

Seropositivity (n,%) : number and percentage of subjects with titer ≥ 10
Seroprotection (n,%) : number and percentage of subjects with titer ≥ 40
Seroconversion (n,%) : number and percentage of subjects with at least a 4-fold increase in titres from day 0 to day 21

In all cases, the conversion factor (fold increase in serum HI GMTs after vaccination) was greater than 2.5. the level required for a successful influenza vaccine.

The percentage of subjects with a two-fold or a four-fold increase in the specific/total mucosal IgA antibody ratio between day 21 and day 0 (1 dose) can be seen in Table 3.

**Table 3:**

Percentages of subjects with a two-fold or a four-fold increase in the specific/total IgA ratio between day 21 and day 0 (1 dose).

| **Strain** | **Group** | **N** | **2 fold increase (%)** | **4 fold increase (%)** |
|---|---|---|---|---|
| **A/Beijing** | Tween & Triton | 20 | 55.0 | 30.0 |
| | Fluarix™ | 19 | 52.6 | 26.3 |
| **A/Sydney** | Tween & Triton | 20 | 65.0 | 45.0 |
| | Fluarix™ | 19 | 47.4 | 5.3 |
| **B/Harbin** | Tween & Triton | 20 | 40.0 | 30.0 |
| | Fluarix™ | 19 | 26.3 | 5.3 |

Summary

The immunogenicity results tabulated above show that the intranasal formulation produced similar levels of seropositivity, seroconversion and seroprotection to those produced by the conventional parenteral vaccine (Fluarix™) twenty-one days after one dose. The intranasal formulation produced a better mucosal IgA response after one dose than the conventional parenteral vaccine (Fluarix™).

**Example 5 - A comparison of the immunogenicity of an intranasal split influenza vaccine formulated with laureth 9, Triton X-100 and Tween 80, with the immunogenicity of a licensed conventional parenteral vaccine (Fluarix™) in healthy adult subjects.**

An intranasal formulation of egg-derived split influenza antigens, formulated with laureth 9. Triton X-100 and Tween 80 (A) was evaluated and compared with Fluarix™/$\alpha$-Rix® (B). The formulations contained three inactivated split virion antigens prepared from the WHO recommended strains of the 1998/1999 season. The device used for administration of the vaccines was the Accuspray™ intranasal syringe from Becton Dickinson. The device works on a similar basis to a conventional syringe, but has a special tip containing spiral channels which result in the production of a spray when even pressure is exerted on the plunger. 100$\mu$l of the formulation was sprayed in each nostril.

Composition of the formulation

The intranasal formulation (A) contained the following inactivated split virions:

1. 30μg HA A/beijing/262/95 (H1N1)
2. 30μg HA A/Sydney/5/97 (H3N2)
3. 30μg HA of B/Harbin/7/94

and phosphate buffered saline pH 7.4± 0.1, Tween 80 0.1%, Triton X-100 0.015% , sodium deoxycholate 0.0045% and thiomersal below 35μg/ml.

The volume of one dose was 200μl (100μl sub-doses for each nostril). Formulation A was formulated with laureth 9 to obtain a final concentration of 0.5% (w/v).

The comparator Fluarix™/α-Rix® (B) is SmithKlineBeecham Biologicals' commercial inactivated trivalent split influenza vaccine, which is administered intramuscularly in a dose of 500μl.

Immunogenicity Study

An open, controlled and randomised study evaluated the immunogenicity of an intranasal split influenza vaccine formulated with laureth 9 supplemented with Tween 80 and Triton X-100 compared to the conventional parenteral vaccine (i.e. Fluarix™). Twenty healthy adult subjects (aged 18-40 years) received one dose of Fluarix™ and ten subjects received one dose (two sub-doses, one per nostril) of the intranasal influenza vaccine.

There was an eight-day follow-up period for solicited local and general symptoms and both vaccines were well-tolerated in relation to safety and reactogenicity. No serious adverse events related to vaccination were reported.

The immunogenicity of the vaccines was examined by assessing the serum haemagglutination inhibition (HI) titres to determine seroconversion rate (defined as the percentage of vaccinees who have at least a 4-fold increase in serum HI titres on day 21 compared to day 0, for each vaccine strain), conversion factor (defined as the fold increase in serum HI Geometric Mean Titres (GMTs) on day 21 compared to day 0, for each vaccine strain) and seroprotection rate (defined as the percentage of vaccinees with a serum HI titre ≥40 after vaccination (for each vaccine strain) that is accepted as indicating protection). In addition, the mucosal IgA antibody response was assessed by Enzyme Linked Immunosorbent Assay (ELISA).

HI seropositivity, serconversion and seroprotection rates twenty-one days after one dose of Fluarix™ or the intranasal formulation can be seen in Table 4.

**Table 4: HI** seropositivity, serconversion and seroprotection rates at 21 days post dose 1:

| Strain | Group | Timing | N | Seropositivity | | Seroprotection | | Seroconversion | |
|---|---|---|---|---|---|---|---|---|---|
| | | | | n | % | n | % | n | % |
| **A/Beijing** | Intranasal vaccine plus Laureth 9 | Day 0 | 20 | 5 | 25.0 | 1 | 5.0 | | |
| | | Day 21 | 20 | 19 | 95.0 | 10 | 50.0 | 15 | 75.0 |
| | Fluarix™ | Day 0 | 19 | 4 | 21.1 | 3 | 15.8 | | |
| | | Day 21 | 19 | 19 | 100.0 | 18 | 94.7 | 19 | 100.0 |
| **A/Sydney** | Intranasal vaccine plus Laureth-9 | Day 0 | 20 | 16 | 80.0 | 4 | 20.0 | | |
| | | Day 21 | 20 | 20 | 100.0 | 19 | 95.0 | 15 | 75.0 |
| | Fluarix™ | Day 0 | 19 | 14 | 73.7 | 1 | 5.3 | | |
| | | Day 21 | 19 | 19 | 100.0 | 18 | 94.7 | 16 | 84.2 |

(continued)

| Strain | Group | Timing | N | Seropositivity | | Seroprotection | | Seroconversion | |
|---|---|---|---|---|---|---|---|---|---|
| | | | | n | % | n | % | n | % |
| B/Harbin | Intranasal vaccine plus Laureth-9 | Day 0 | 20 | 18 | 90.0 | 11 | 55.0 | | |
| | | Day 21 | 20 | 20 | 100.0 | 19 | 95.0 | 12 | 60.0 |
| | Fluarix™ | Day 0 | 19 | 17 | 89.5 | 11 | 57.9 | | |
| | | Day 21 | 19 | 19 | 100.0 | 19 | 100,0 | 15 | 78.9 |

Seropositivity (n,%) : number and percentage of subjects with titer ≥ 10 Seroprotection (n,%) : number and percentage of subjects with titer ≥ 40 Seroconversion (n,%) : number and percentage of subjects with at least a 4-fold increase in titres from day 0 to day 21

In all cases, the conversion factor (fold increase in serum HI GMTs after vaccination) was greater than 2.5, the level required for a successful influenza vaccine.

The percentage of subjects with a two-fold or a four-fold increase in the specific/total mucosal IgA antibody ratio between day 21 and day 0 (1 dose) can be seen in Table 5.

**Table 5:** Percentages of subjects with a two-fold or a four-fold increase in the specific/total IgA ratio between day 21 and day 0 (1 dose).

| Strain | Group | N | 2 fold increase (%) | 4 fold increase (%) |
|---|---|---|---|---|
| A/Beijing | Laureth-9 | 20 | 50.0 | 20.0 |
| | Fluarix™ | 19 | 52.6 | 26.3 |
| A/Sydney | Laureth-9 | 20 | 55.0 | 25.0 |
| | Fluarix™ | 19 | 47.4 | 5.3 |
| B/Harbin | Laureth-9 | 20 | 15.0 | 10.0 |
| | Fluarix™ | 19 | 26.3 | 5.3 |

Summary

The immunogenicity results tabulated above show that the intranasal formulation produced similar levels of seropositivity, seroconversion and seroprotection to the conventional parenteral vaccine (Fluarix™) twenty-one days after one dose. The intranasal formulation generally produced a better mucosal IgA response after one dose than the conventional parenteral vaccine (Fluarix™).

**Example 6 - Evaluation of an intranasal flu vaccine with and without laureth 9 + 3D-MPL in primed mice.**

**6.1**. In a first experiment, mice were vaccinated with candidate formulations containing the same influenza strains as those used for their priming.

**Experimental procedure**

Female Balb/c mice (8 weeks old) were "primed" intranasally at day 0 with β-propiolactone inactivated egg-derived trivalent whole influenza virus A/Beijing/262/95. A/Sydney/5/97 and B/Harbin/7/94; 5 μg HA/strain) so as to mimic natural priming which occurs in humans.

After 28 days, mice (10 animals per group) were intranasally vaccinated with the following trivalent vaccine formulations containing the same strains as those used for the priming:

| Group | Route (Method) | Trivalent split antigens | additional reagents? |
|---|---|---|---|
| Plain 1 | Intranasal (droplets) | 3.0 μg HA/strain | no |

(continued)

| Group | Route (Method) | Trivalent split antigens | additional reagents? |
|---|---|---|---|
| Plain 2 | Intranasal (droplets) | 1.5 $\mu$g HA/strain | no |
| L9 | Intranasal (droplets) | 1.5 $\mu$g HA/strain | 0.5 % Laureth-9 |
| L9 + MPL | Intranasal (droplets) | 0.75 $\mu$g HA/strain | 0.5 % Laureth-9 + 5 $\mu$g MPL |
| Parenteral | Intramuscular (injection) | 1.5 $\mu$g HA/strain | no |

Intranasal vaccine formulations administered to mice are similar to those that are administered to humans in Example 7 except that the administered dose for mice corresponds to 1/10th of the human dose.

Serum samples were collected at day 42 and tested for haemagglutination inhibition (HI) antibodies. Following sacrifice (day 42), nasal washings were performed and tested for IgA antibody titers by ELISA. Specific IgA antibodies were measured as end point titers (EPT) and the results were expressed as specific IgA EPT per $\mu$g total IgA in order to exclude any difference due to the sampling method.

**Results**

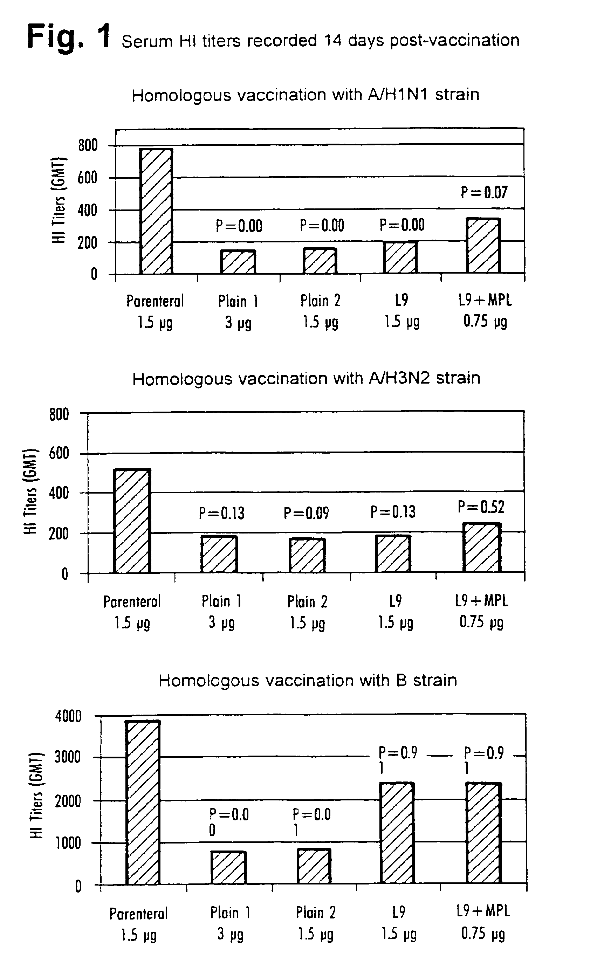

The first objective of the study was to confirm that intranasal vaccine formulations are capable of eliciting serum HI titers not significantly different from those resulting from parenteral administration. Figure 1 shows the HI titers observed in serum at day 42 (i.e. 14 days post- vaccination) with the various vaccines.

Statistical analysis (Tukey-HSD statistical comparative assay) was performed on the observed HI titers in order to compare the intranasal vaccines to the parenteral vaccine. The HI titers observed with Plain 1 and 2 groups were significantly different ($p < 0.05$) from those induced by the parenteral vaccine for two of the three Flu strains (A/H1N1 and B strains). The anti-A/H3N2 strain titers were not significantly different. The L9 formulation was as immunogenic as the parenteral vaccine for two out of three strains (A/H3N2 and B strains). In contrast, the L9 + MPL formulated vaccine elicited HI antibodies against the three Flu strains with titers which did not differ significantly from those observed with the parenteral vaccine.

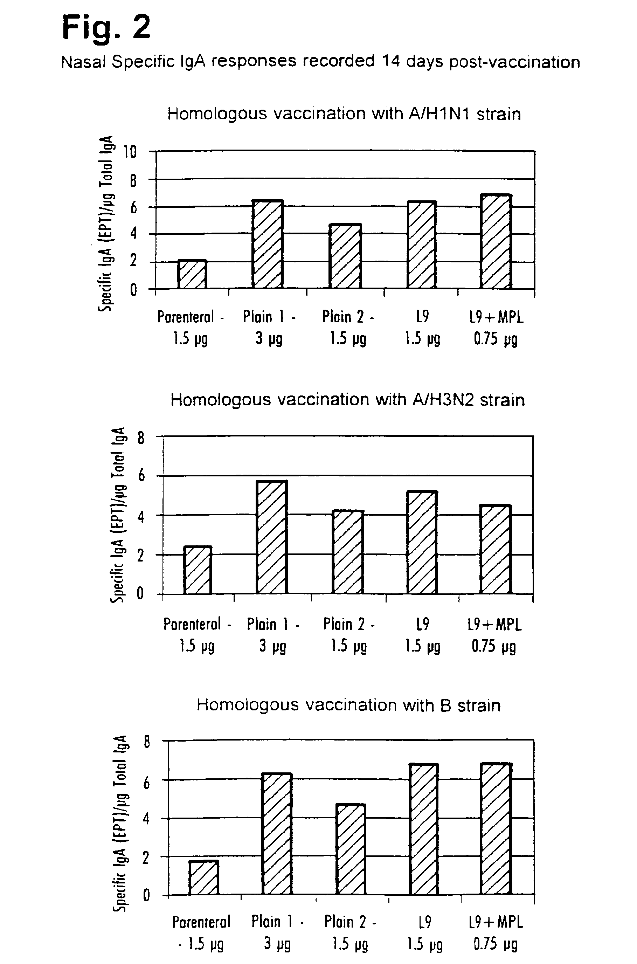

The second objective was to determine whether or not the nasal IgA response to intranasal vaccination was superior to that observed in animals boosted intramuscularly. Figure 2 presents the nasal IgA response recorded 14 days after the booster vaccination (day 42).

There were no significant differences between the responses induced by any of the intranasal formulations. Intranasal administration elicited two to four-fold higher responses compared to the parenteral route. Finally, the vaccine formulated with L9 + MPL was able to sustain the same level of response compared to the other intranasal vaccines but with a lower antigen dose.

**Conclusions**

Trivalent split influenza antigens formulated with L9 + MPL (0.75 $\mu$g HA + 0.5 % Laureth 9 + 5 $\mu$g MPL) were the most immunogenic intranasal vaccine formulation in terms of HI antibody response.

All nasally delivered vaccines tested were more potent in inducing local 1gA antibodies than the parenterally administered vaccine. The L9 + MPL formulation induced a similar response to the other formulations but with a reduced antigen content.

**6.2** In a second experiment, mice were immunised intranasally with strains which were different to those used for priming.

Antigenic drift is responsible for annual epidemics. The strains included every year in the Flu vaccine are those found most currently; yet other strains, more or less related, may also be found in the field. As a consequence, the best candidate Flu vaccine will have to induce protection against a broad range of strains to be efficient. Therefore, it was of interest to investigate to which extent a given intranasal formulation was capable of eliciting an immune response after a "priming" with strains heterologous to those contained in the vaccine.

**Experimental procedure**

Female Balb/c mice (8 weeks old) were "primed" intranasally at day 0 with $\beta$-propiolactone inactivated egg-derived trivalent whole influenza virus (A/Johannesburg/82/96 H1N1, A/Johannesburg/33/94 H3N2 and B/Panama/45/90; 5 $\mu$g HA / strain) to mimic natural priming which occurs in humans.

After 28 days, the mice (10 animals per group) were intranasally vaccinated with the following trivalent vaccine formulations (containing A/Beijing/262/95 H1N1, A/Sydney/5/97 H3N2 and B/Harbin/7/94 as heterologous strains).

| Group | Route (Method) | Trivalent split antigens | additional reagent? |
|---|---|---|---|
| Plain 1 | Intranasal (droplets) | 3.0 µg HA/strain | no |
| Plain 2 | Intranasal (droplets) | 1.5 µg HA/strain | no |
| L9 | Intranasal (droplets) | 1.5 µg HA/strain | 0.5 % Laureth-9 |
| L9 + MPL | Intranasal (droplets) | 0.75 µg HA/strain | 0.5 % Laureth-9 + 5 µg MPL |
| Parenteral | Intramuscular (injection) | 1.5 µg HA/strain | no |

The amounts of trivalent split virions contained in the various intranasal vaccine formulations to be tested on mice again correspond to 1/10th of the dose to be administered to human volunteers in Example 7.

Serum samples were collected at day 42 and tested for haemagglutination inhibition (HI) antibodies. Following sacrifice (day 42), nasal washings were performed and tested for IgA antibody titers by ELISA. Specific IgA antibodies were measured as end point titers (EPT) and the results were expressed as specific IgA EPT per µg total IgA in order to exclude any difference due to the sampling method.

## Results

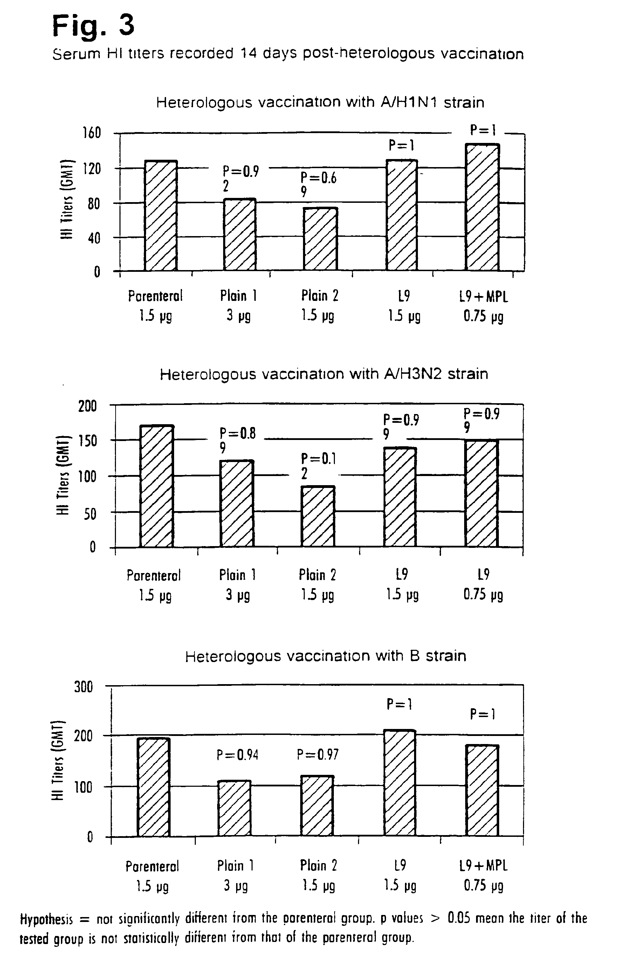

The first objective of the study was to determine if the intranasal vaccine formulations were capable of eliciting serum HI responses against vaccine antigens when heterosubtypic strains are used for priming. Figure 3 shows the HI titers observed in serum 14 days post-vaccination (day 42) with the various vaccine formulations.

All intranasal formulations with L9 or L9 + MPL were able to induce immune responses directed towards the three influenza strains, which were comparable to those elicited by parenteral administration of the plain vaccine. Statistical analysis (Tukey-HSD statistical comparative assay) confirmed that responses elicited by all intranasal formulations were not significantly ($p > 0.05$) different from parenteral administration responses. Within intranasal formulations, no statistical differences were observed. Yet the L9 + MPL and L9 formulations were generally more immunogenic, the former containing half the latter antigen content (0.75 µg versus 1.5 µg HA).

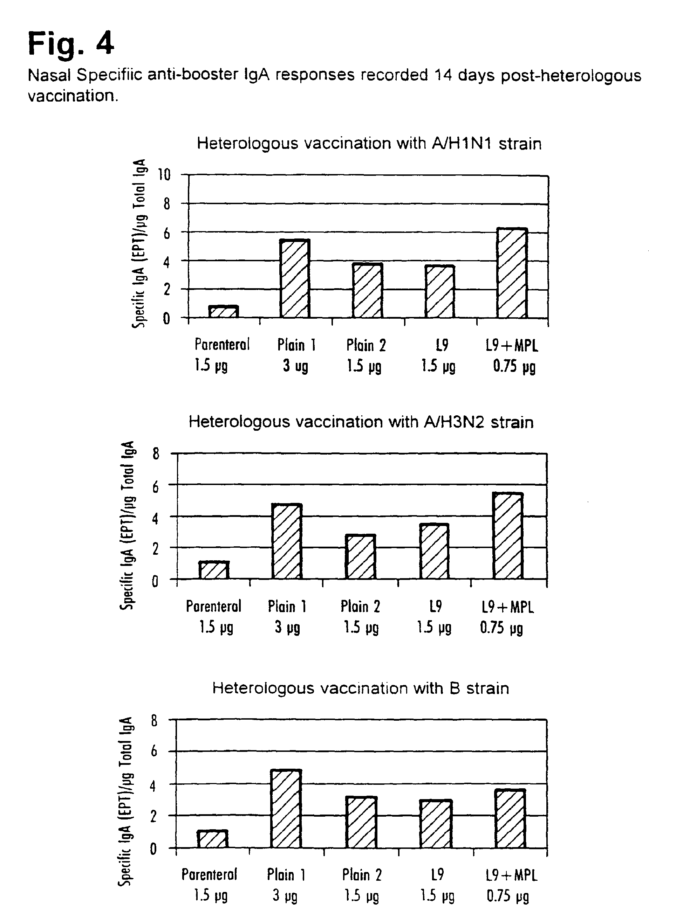

The second objective was to determine (1) if a nasal specific IgA response to heterologous strains was measurable after intranasal vaccination and (2) if this response was superior to that observed in animals vaccinated intramuscularly. Figure 4 presents the nasal specific anti-heterologous IgA response recorded 14 days post-vaccination (day 42).

The criteria of the second objective were both met. All intranasal formulations induced IgA responses towards the three heterologous strains that were three- to eight-fold higher than those observed when plain vaccine was injected intramuscularly. The amplitude of the IgA responses was not significantly different within the intranasal formulations.

The magnitude of the responses observed with the heterologous vaccination reached the same range as for the homologous vaccination (see Figure 2). Here again, L9 + MPL formulated vaccine was able to sustain the same amplitude of response with a lower dose of antigen (0.75 µg HA) in comparison with the plain or L9 formulations (3 and 1.5 µg HA).

## Conclusion

Intranasal administration of trivalent split influenza vaccine, with or without absorption-enhancing surfactant or adadjuvantation, was able to induce heterosubtypic responses both in terms of serum HI antibody and intra-nasal IgA specific for Flu vaccine strains.

Although the differences between groups were not statistically significant, vaccines where the trivalent split virions are formulated with L9 or L9 + MPL generally induced a more potent systemic as well as local immune response. In addition, compared to L9 containing vaccine, the vaccine formulated with L9 + MPL induced the same level of immunity but with a lower antigen dosage.

## Example 7 - Evaluation of an intranasally administered egg-derived trivalent split virion influenza vaccine with or without laureth 9 or laureth 9 + 3D-MPL administered following a one dose schedule, compared to an intramuscularly administered trivalent split virion influenza vaccine in healthy adults from 18-40 years of age.

In this study the local mucosal and systemic immune responses to the vaccines are evaluated in approximately 120 healthy male and female subjects.

**Composition of the candidate vaccines**

Five formulations of the egg-derived split influenza antigens are evaluated in this study. The candidate vaccines are administered intranasally. In addition, SmithKline Beecham Biologicals' Fluarix™ - inactivated split virion influenza vaccine administered intramuscularly - is used as a comparator.

The candidate intranasal vaccines contain the three inactivated split virion antigens used in the formulation of Fluarix™. The strains are the ones that have been recommended by the WHO for the 2000 Southern Hemisphere season. A general description of the various formulations is presented in Table 6.

**Table 6** : General description of the vaccines

| Administration | Antigen per dose (μg HA/strain) | Additional Reagent? |
|---|---|---|
| intranasal | 30 μg | no |
| intranasal | 15 μg | no |
| intranasal | 15 μg | Laureth-9 |
| intranasal | 7.5 μg | Laureth-9 |
| intranasal | 7.5 μg | Laureth-9 + 3D- MPL |
| intramuscular | 15 μg | no |

**Intranasal trivalent split influenza vaccines without L9 or MPL (30 μg dose and 15 μg dose)**

The volume of one dose is 0.2 ml.

Table 7:

| Component | Quantity per dose* |
|---|---|
| Inactivated split virions<br>- A/New Caledonia/20/99 (H1N1)<br>- A/Sydney/5/97 (H3N2)<br>- B/Yamanashi/166/98 | <br>30 or 15 μg HA<br>30 or 15 μg HA<br>30 or 15 μg HA |
| Phosphate buffered saline<br>(pH 7.0-7.4)<br>- Anhydrous dibasic sodium phosphate<br>- Monobasic potassium phosphate<br>- Potassium chloride<br>- Sodium chloride<br>Triton X100<br>Tween 80<br>Water for injection | <br><br>8.10 mM<br><br>1.47 mM<br><br>2.70 mM<br>137 mM<br>0.02 %<br>0.15 %<br>q.s. ad 0.2 ml |
| Residual thiomersal | < 2 μg |
| *  Each intranasal vial contains a 20 % volume overage | |

**Intranasal trivalent split influenza vaccines formulated with L9 (15 μg dose and 7.5 μg dose)**

The volume of one dose is 0.2 ml. The formulation is as shown in Table 7. further including 1 mg of laureth 9 per dose, together with a 15 or 7.5 μg dose of HA per strain. Laureth 9 is obtained from Kreussler, Germany).

**Intranasal trivalent split influenza vaccine formulated with L9 + 3D-MPL (7.5 μg dose)**

The volume of one dose is 0.2 ml. The formulation is as shown in Table 7, further including 1 mg of laureth 9 and 50 μg of 3D-MPL per dose, together with a dose of 7.5 μg HA per strain.

**The Fluarix™ commercial inactivated trivalent split influenza vaccine used as comparator**

Fluarix™ 2000 (Southern Hemisphere), is used as comparator. This 0.5 ml dose vaccine is administered intramuscularly.

One dose contains 15 μg haemagglutinin of each influenza virus strain (A/New Caledonia/20/99 (H1N1) - A/Sydney/5/97 (H3N2) - B/Yamanashi/166/98) and 50 μg of thiomersal as preservative per dose.

**Formulation of the trivalent split influenza candidate vaccines**

*1. Concentration of the three inactivated split virions*

Before formulation of the final candidate vaccines, the three inactivated split virions were concentrated separately by tangential flow filtration up to 1000 to 1500 μg of HA per ml. Membrane cassettes equipped with a cellulose triacetate membrane with a cut-off of 10 kDa were used.

*2. Formulation of the trivalent split influenza vaccines*

The formulation flow diagram is presented below:

Water for injection

+

Phosphate buffer saline pH 7.4

+

Tween 80 to 0.15 %

+

Triton X100 to 0.02 %

+                              → *stirring 5 min. at room temperature*

Strain A/ New Caledonia/20/99
150 or 75 or 37.5 μg HA/ml


+                              → *stirring 10 min. at room temperature*

Strain A/Sydney/5/97
150 or 75 μg HA/ml

+                              → *stirring 10 min. at room temperature*

Strain B/ Yamanashi/166/98
150 or 75μg HA/ml

+                              → *stirring 15 min. at room temperature*

adjust pH to 7.2 ± 0.2

↓

| Final bulk |              → *stored at +2 to +8 °C*

For the laureth 9-containing formulations, laureth-9 to 0.5% is added immediately before the pH adjustment and stirring is continued at room temperature for 15 minutes.

For the laureth 9 + 3D-MPL-containing formulations, 250 $\mu$g/ml 3D-MPL is added immediately prior to the addition of the laureth 9 and the formulation is stirred for 15 minutes before the laureth 9 is added.

**Filling of the trivalent split influenza candidate vaccines**

The final bulks are aseptically filled in type-1 (Ph. Eur.) glass vials from Pfeiffer (Germany). Immediately after filling, these vials are closed with a rubber stopper. All operations are performed in an aseptic room (laminar flow system).

After filling and closing, the stoppered vials are inserted into a plastic plunger and assembled into a spraying nozzle device for spray generation. This device allows the administration of two sprays of 100 $\mu$l.

**Results**

For all subjects the following analysis is carried out:

1. At days 0. 21 & 42: mucosal IgA (local immune response) ELISA titres, tested separately against each of the three influenza virus strains represented in the vaccine.
2. At days 0, 21 & 42: serum haemagglueination-inhibition (HI) antibody titres, tested separately against each of the three influenza strains.

Derived from these are:

1. Serum HI antibody GMTs (with 95% confidence intervals) at all time points.
2. For HI: seroconversion rates at day 21.
3. For HI: conversion factors at day 21.
4. For HI: protection rates at days 21 & 42.
5. For IgA only: percentage of subjects who have a 2-fold and 4-fold increase in IgA titres from day 0 to day 21 and day 0 to day 42.

**Claims**

**1.** The use of a non-live split influenza virus antigen preparation in the manufacture of a vaccine formulation for a one-dose intranasal vaccination against influenza, wherein the one-dose vaccination achieves at least two out of the following criteria for the or all strains of influenza present in the vaccine:

(i) a seroconversion rate of greater than or equal to 40% for a human adult population (18-60 years) or to 30% in a human elderly population (>60 years);
(ii) a seroprotection rate of greater than or equal to 70% for a human adult population (18-60 years) or to 60% in a human elderly population (>60 years); and
(iii) a conversion factor of greater than or equal to 2.5 for a human adult population (18-60 years) or to 2.0 in a human elderly population (>60 years)..

**2.** The use according to claim 1 wherein all three of the European Union criteria as set forth in claim 1 are met for the or all strains of influenza represented in the vaccine.

**3.** The use according to claim 1 or 2 wherein the formulation comprises at least one surfactant.

**4.** The use according to claim 3 wherein the surfactant is at least one non-ionic surfactant selected from the group consisting of the octylphenoxypolyethoxyethanols (for example from the commercially available Triton ™ series), polyoxyethylene sorbitan esters (Tween ™ series) and polyoxythylene ethers or esters of general formula (I):

$$\text{(I)} \qquad \text{HO(CH}_2\text{CH}_2\text{O)}_n\text{-A-R}$$

wherein n is 1-50, A is a bond or -C(O)-, R is $C_{1-50}$ alkyl or phenyl $C_{1-50}$ alkyl, and combinations of two or more of these.

**5.** The use according to claim 4 wherein the non-ionic surfactant is at least one surfactant selected from the group consisting of t-octylphenoxypolyethoxyethanol (Triton X-100), polyoxyethylene sorbitan monooleate (Tween 80) and laureth 9, or a combination of two or more of these.

6. The use according to claim 5 wherein the vaccine comprises a combination of two of the three non-ionic surfactants, namely polyoxyethylene sorbitan monooleate (Tween 80) and t-octylphenoxypolyethoxyethanol (Triton X-100).

7. The use according to claim 6 wherein the vaccine comprises a combination of all three non-ionic surfactants.

8. The use according to any one of claims 1 to 7 wherein the vaccine further comprises a bile acid or cholic acid, or derivative thereof such as sodium deoxycholate.

9. The use according to any one of claims 1 to 8 wherein each dose of the vaccine formulation contains a low dose of haemagglutinin.

10. The use according to claim 9 wherein the haemagglutinin content per influenza strain is 30 $\mu$g or less per dose.

11. The use according to claim 10 wherein the haemagglutinin content per influenza strain is 15 $\mu$g or less per dose.

12. The use according to claim 11 in which the heamagglutinin content is 7.5 $\mu$g or less of haemagglutinin per virus strain per vaccine dose.

13. The use according to any one of claims 1 to 12 wherein the vaccine formulation is in a volume per dose which is less than 500 $\mu$l.

14. The use according to claim 13 wherein the volume per dose is less than 300 $\mu$l or not more than 200 $\mu$l per dose.

15. The use according to any one of claims 1 to 14 wherein the vaccine is delivered in a bi-dose format of two sub-doses.

16. The use according to any one of claims 1 to 15, wherein the vaccine does not contain an added immunostimulant.

17. The use according to any one of claims 1 to 16, wherein the vaccine further comprises a non-toxic derivative of lipid A, preferably selected from non-toxic derivatives of monophosphoryl lipid A and diphosphoryl lipid A.

18. The use according to claim 17, wherein the vaccine comprises 3-de-O-acylated monophosphoryl lipid A (3D-MPL).

19. The use according to claim 18, wherein the vaccine comprises 3-de-O-acylated monophosphoryl lipid A (3D-MPL) and laureth 9.

20. A pharmaceutical kit comprising an intranasal delivery device and a one-dose vaccine which comprises a non-live split influenza virus antigen preparation without an added immunostimulant, which generates an immune response that achieves at least two out of the criteria as set forth in claim 1 for the or all strains of influenza present in the vaccine.

21. A pharmaceutical kit comprising an intranasal delivery device and a one-dose split influenza vaccine which generates an immune response that achieves at least two out of the criteria as set forth in claim 1 for the or all strains of influenza present in the vaccine.

22. A pharmaceutical kit comprising an intranasal delivery device and a one-dose vaccine which comprises a low HA dose of a non-live split influenza virus antigen preparation which generates an immune response that achieves at least two out of the criteria as set forth in claim 1 for the or all strains of influenza present in the vaccine.

23. The pharmaceutical kit according to any one of claims 20 to 22 wherein the device is a bi-dose delivery device for delivering two sub-doses in a single administration.

24. The pharmaceutical kit according to any one of claims 20 to 23 wherein the device is an intranasal spray device.

25. A method of manufacturing a one-dose influenza vaccine for nasal application which method comprises:

(i) providing a split influenza virus preparation produced essentially as for a conventional injected influenza vaccine and comprising at least one non-ionic surfactant;
(ii) optionally adjusting the concentration of the haemagglutinin and/or the concentration of non-ionic surfactant in the preparation;
(iii) filling an intranasal delivery device with a vaccine dose from the split influenza virus preparation, said dose being a suitable volume for intranasal administration, optionally in a bi-dose format, and which one dose application generates an immune response that achieves at least two of the criteria as set forth in claim 1 for the or all strains of influenza present in the vaccine.

**Patentansprüche**

1. Verwendung einer nicht-lebenden Split-Influenzavirus-Antigenzubereitung bei der Herstellung einer Impfstoffformulierung für eine intranasale Einzeldosisimpfung gegen Influenza, worin die Einzeldosisimpfung wenigstens zwei der folgenden Kriterien für den oder alle im Impfstoff vorkommenden Stämme von Influenza erreicht:

   (i) eine Serokonversionsrate von größer als oder gleich 40 % für eine Population erwachsener Menschen (18-60 Jahre) oder 30 % in einer Population älterer Menschen (>60 Jahre);
   (ii) eine Seroprotektionsrate von größer als oder gleich 70 % für eine Population erwachsener Menschen (18-60 Jahre) oder 60 % in einer Population älterer Menschen (>60 Jahre); und
   (iii) ein Konversionsfaktor von größer als oder gleich 2,5 für eine Population erwachsener Menschen (18-60 Jahre) oder 2,0 in einer Population älterer Menschen (>60 Jahre).

2. Verwendung gemäß Anspruch 1, worin alle drei der Kriterien der Europäischen Union, wie in Anspruch 1 dargelegt, für den oder alle im Impfstoff vertretenen Stämme von Influenza erfüllt sind.

3. Verwendung gemäß Anspruch 1 oder 2, worin die Formulierung wenigstens ein Tensid umfaßt.

4. Verwendung gemäß Anspruch 3, worin das Tensid wenigstens ein nicht-ionisches Tensid ist, das aus der Gruppe ausgewählt ist, die aus folgenden besteht: Octylphenoxypolyethoxyethanole (zum Beispiel aus der kommerziell erhältlichen Triton™-Reihe), Polyoxyethylensorbitanester (Tween™-Reihe) und Polyoxyethylenether oder -ester der allgemeinen Formel (I) :

$$HO(CH_2CH_2O)_n\text{-}A\text{-}R \qquad (I)$$

   worin n 1 bis 50 ist, A eine Bindung oder -C(O)- ist, R $C_{1-50}$-Alkyl oder Phenyl-$C_{1-50}$-alkyl ist, und Kombinationen aus zwei oder mehreren daraus.

5. Verwendung gemäß Anspruch 4, worin das nicht-ionische Tensid wenigstens ein Tensid ist, das aus der Gruppe ausgewählt ist, die aus t-Octylphenoxypolyethoxyethanol (Triton X-100), Polyoxyethylensorbitanmonooleat (Tween 80) und Laureth 9 besteht, oder eine Kombination von zwei oder mehreren daraus ist.

6. Verwendung gemäß Anspruch 5, worin der Impfstoff eine Kombination von zwei der drei nicht-ionischen Tenside umfaßt, nämlich Polyoxyethylensorbitanmonooleat (Tween 80) und t-Octylphenoxypolyethoxyethanol (Triton X-100).

7. Verwendung gemäß Anspruch 6, worin der Impfstoff eine Kombination von allen der drei nicht-ionischen Tenside umfaßt.

8. Verwendung gemäß einem der Ansprüche 1 bis 7, worin der Impfstoff ferner eine Gallensäure oder Cholsäure oder ein Derivat davon, wie Natriumdesoxycholat, umfaßt.

9. Verwendung gemäß einem der Ansprüche 1 bis 8, worin jede Dosis der Impfstoffformulierung eine niedrige Dosis an Hämagglutinin enthält.

10. Verwendung gemäß Anspruch 9, worin der Hämagglutinin-Gehalt pro Influenzastamm 30 $\mu$g oder weniger pro Dosis ist.

11. Verwendung gemäß Anspruch 10, worin der Hämagglutinin-Gehalt pro Influenzastamm 15 $\mu$g oder weniger pro

Dosis ist.

**12.** Verwendung gemäß Anspruch 11, worin der Hämagglutinin-Gehalt 7,5 µg oder weniger an Hämagglutinin pro Virusstamm pro Impfstoffdosis ist.

**13.** Verwendung gemäß einem der Ansprüche 1 bis 12, worin die Impfstoffformulierung in einem Volumen pro Dosis ist, das weniger als 500 µl ist.

**14.** Verwendung gemäß Anspruch 13, worin das Volumen pro Dosis weniger als 300 µl oder nicht mehr als 200 µl pro Dosis ist.

**15.** Verwendung gemäß einem der Ansprüche 1 bis 14, worin der Impfstoff in einem Bi-Dosisformat von zwei Unterdosen zugeführt wird.

**16.** Verwendung gemäß einem der Ansprüche 1 bis 15, worin der Impfstoff kein zugegebenes Immunstimulans enthält.

**17.** Verwendung gemäß einem der Ansprüche 1 bis 16, worin der Impfstoff ferner ein nicht-toxisches Derivat von Lipid A umfaßt, das vorzugsweise ausgewählt ist aus nicht-toxischen Derivaten von Monophosphoryl-Lipid A oder Diphosphoryl-Lipid A.

**18.** Verwendung gemäß Anspruch 17, worin der Impfstoff 3-des-O-acyliertes Monophosphoryl-Lipid A (3D-MPL) umfaßt.

**19.** Verwendung gemäß Anspruch 18, worin der Impfstoff 3-des-O-acyliertes Monophosphoryl-Lipid A (3D-MPL) und Laureth 9 umfaßt.

**20.** Pharmazeutisches Kit, das eine intranasale Zuführungsvorrichtung und einen Einzeldosisimpfstoff umfaßt, der eine nicht-lebende Split-Influenzavirus-Antigenzubereitung ohne ein zugegebenes Immunstimulans umfaßt, wobei der Impfstoff eine Immunantwort erzeugt, die wenigstens zwei der Kriterien, die in Anspruch 1 dargelegt sind, für den oder alle im Impfstoff vorliegenden Stämme von Influenza erreicht.

**21.** Pharmazeutisches Kit, das eine intranasale Zuführungsvorrichtung und einen Einzeldosis-Split-Influenza-Impfstoff umfaßt, der eine Immunantwort erzeugt, die wenigstens zwei der Kriterien, die in Anspruch 1 dargelegt sind, für den oder alle im Impfstoff vorliegenden Stämme von Influenza erreicht.

**22.** Pharmazeutisches Kit, das eine intranasale Zuführungsvorrichtung und einen Einzeldosisimpfstoff umfaßt, der eine niedrige HA-Dosis einer nicht-lebenden Split-Influenzavirus-Antigenzubereitung umfaßt, die eine Immunantwort erzeugt, die wenigstens zwei der Kriterien, die in Anspruch 1 dargelegt sind, für den oder alle im Impfstoff vorliegenden Stämme von Influenza erreicht.

**23.** Pharmazeutisches Kit gemäß einem der Ansprüche 20 bis 22, worin die Vorrichtung eine Vorrichtung zur Bi-Dosiszuführung für die Zuführung von zwei Unterdosen in einer einzelnen Verabreichung ist.

**24.** Pharmazeutisches Kit gemäß einem der Ansprüche 20 bis 23, worin die Vorrichtung eine intranasale Sprühvorrichtung ist.

**25.** Verfahren zur Herstellung eines Einzeldosis-Influenzaimpfstoffs für die nasale Anwendung, wobei das Verfahren folgendes umfaßt:

(i) Bereitstellen einer Split-Influenzavirus-Zubereitung, die im wesentlichen wie für einen herkömmlich injizierten Influenzaimpfstoff hergestellt wurde und wenigstens ein nicht-ionisches Tensid umfaßt;
(ii) optionales Einstellen der Konzentration des Hämagglutinins und/oder der Konzentration des nicht-ionischen Tensids in der Zubereitung;
(iii) Befüllen einer intranasalen Zuführungsvorrichtung mit einer Impfstoffdosis der Split-Influenzavirus-Zubereitung, wobei die Dosis ein geeignetes Volumen für die intranasale Verabreichung, gegebenenfalls in einem Bi-Dosisformat, ist, und wobei die Einzeldosisanwendung eine Immunantwort erzeugt, die wenigstens zwei der Kriterien, wie in Anspruch 1 dargelegt, für den oder alle im Impfstoff vorliegenden Influenzastämme erreicht.

**Revendications**

1. Utilisation d'une préparation d'antigène de virus influenza fragmenté non-vivant dans la fabrication d'une formulation vaccinale pour une vaccination intranasale unidose contre influenza, dans laquelle la vaccination unidose répond à au moins deux des critères suivants pour la ou toutes les souches d'influenza présentées dans le vaccin :

   (i) un taux de séroconversion supérieur ou égal à 40 % pour une population adulte humaine (18-60 ans) ou à 30 % dans une population de personnes âgées (> 60a ans) ;
   (ii) un taux de séroprotection supérieur ou égal à 70 % pour une population adulte humaine (18-60 ans) ou à 60 % dans une population de personnes âgées (> 60 ans) ; et
   (iii) un facteur de conversion supérieur ou égal à 2,5 pour une population adulte humaine (18-60 ans) ou à 2,0 dans une population de personnes âgées (> 60a ans).

2. Utilisation selon la revendication 1 dans laquelle les trois critères de l'Union européenne tels qu'énoncés dans la revendication 1 sont satisfaits pour la ou toutes les souches d'influenza représentées dans le vaccin.

3. Utilisation selon la revendication 1 ou 2 dans laquelle la formulation comprend au moins un surfactant.

4. Utilisation selon la revendication 3 dans laquelle le surfactant est au moins un surfactant non-ionique sélectionné à partir du groupe constitué par les octylphénoxypolyéthoxyéthanols (par exemple des séries Triton™ disponibles dans le commerce), les esters de polyéthylène sorbitan (séries Tween™) et les éthers ou esters de polyoxyéthylène de formule générale (I) :

$$(I) \qquad HO\,(CH_2CH_2O)_n\text{-A-R}$$

   dans laquelle n est 1-50, A est une liaison ou - C(O)-, R est un $C_{1-50}$ alkyle ou phényl $C_{1-50}$ alkyle, et des combinaisons de deux ou plus de ceux-ci.

5. Utilisation selon la revendication 4 dans laquelle le surfactant non-ionique est au moins un surfactant sélectionné dans le groupe constitué par t-octylphénoxypolyéthoxyéthanol (Triton X-100), monooléate de polyoxyéthylène sorbitan (Tween 80) et laureth 9, ou une combinaison de deux ou plus de ceux-ci.

6. Utilisation selon la revendication 5 dans laquelle le vaccin comprend une combinaison de deux des trois surfactants non-ioniques, à savoir monooléate de polyoxyéthylène sorbitan (Tween 80) et t-octylphénoxypolyéthoxyéthanol (Triton X-100).

7. Utilisation selon la revendication 6 dans laquelle le vaccin comprend une combinaison des trois surfactants non-ioniques.

8. Utilisation selon l'une quelconque des revendications 1 à 7 dans laquelle le vaccin comprend en outre un acide biliaire ou un acide cholique, ou un dérivé de celui-ci comme le déoxycholate de sodium.

9. Utilisation selon l'une quelconque des revendications 1 à 8 dans laquelle chaque dose de la formulation vaccinale contient une faible dose d'hémagglutinine.

10. Utilisation selon la revendication 9 dans laquelle la teneur en hémagglutinine par souche d'influenza est de 30 μg ou moins par dose.

11. Utilisation selon la revendication 10 dans laquelle la teneur en hémagglutinine par souche d'influenza est de 15 μg ou moins par dose.

12. Utilisation selon la revendication 11 dans laquelle la teneur en hémagglutinine est de 7,5 μg ou moins d'hémagglutinine par souche virale par dose de vaccin.

13. Utilisation selon l'une quelconque des revendications 1 à 12 dans laquelle la formulation vaccinale est dans un volume par dose qui est inférieur à 500 μl.

14. Utilisation selon la revendication 13 dans laquelle le volume par dose est inférieur à 300 μl ou pas plus de 200 μl

par dose.

**15.** Utilisation selon l'une quelconque des revendications 1 à 14 dans laquelle le vaccin est délivré dans un format bi-dose de deux sous-doses.

**16.** Utilisation selon l'une quelconque des revendications 1 à 15, dans laquelle le vaccin ne contient pas d'immunostimulant ajouté.

**17.** Utilisation selon l'une quelconque des revendications 1 à 16 dans laquelle le vaccin comprend en outre un dérivé non-toxique de lipide A, de préférence sélectionné à partir des dérivés non-toxiques de monophosphoryl lipide A et de diphosphoryl lipide A.

**18.** Utilisation selon la revendication 17 dans laquelle le vaccin comprend du monophosphoryl lipid A 3-de-O acétylé (3D-MPL).

**19.** Utilisation selon la revendication 18, dans laquelle le vaccin comprend du monophosphoryl lipid A 3-de-O acétylé (3D-MPL) et du laureth 9.

**20.** Kit pharmaceutique comprenant un dispositif de délivrance intranasale et un vaccin unidose qui comprend une préparation d'antigène de virus influenza fragmenté non-vivant sans immunostimulant ajouté, qui entraîne une réponse immunitaire qui répond à au moins deux des critères énoncés dans la revendication 1 pour la ou toutes les souches d'influenza présentes dans le vaccin.

**21.** Kit pharmaceutique comprenant un dispositif de délivrance intranasale et un vaccin unidose à influenza fragmenté qui entraîne une réponse immunitaire qui répond à au moins deux des critères énoncés dans la revendication 1 pour la ou toutes les souches d'influenza présentes dans le vaccin.

**22.** Kit pharmaceutique comprenant un dispositif de délivrance intranasale et un vaccin unidose qui comprend une faible dose HA d'une préparation d'antigène de virus influenza fragmenté non-vivant qui entraîne une réponse immunitaire qui répond à au moins deux des critères énoncés dans la revendication 1 pour la ou toutes les souches d'influenza présentes dans le vaccin.

**23.** Kit pharmaceutique selon l'une quelconque des revendications 20 à 22 dans lequel le dispositif est un dispositif de délivrance bi-dose pour délivrer deux sous-doses en une seule administration.

**24.** Kit pharmaceutique selon l'une quelconque des revendications 20 à 23 dans lequel le dispositif est un dispositif pulvérisateur intranasal.

**25.** Procédé de fabrication d'un vaccin à influenza unidose pour l'application nasale, lequel procédé comprend les étapes suivantes :

(i) fournir une préparation de virus influenza fragmenté produite essentiellement comme pour un vaccin à influenza injecté conventionnel et comprenant au moins un surfactant non-ionique ;
(ii) optionnellement ajuster la concentration de l'hémagglutinine et/ou la concentration de surfactant non-ionique dans la préparation ;
(iii) remplir un dispositif de délivrance intranasale avec une dose de vaccin de la préparation du virus influenza fragmenté, ladite dose étant un volume adaptable pour l'administration intranasale, optionnellement dans un format bi-dose, et dont l'application d'une dose entraîne une réponse immunitaire qui répond à au moins deux des critères énoncés dans la revendication 1 pour la ou toutes les souches d'influenza présentes dans le vaccin.

# Fig. 1 Serum HI titers recorded 14 days post-vaccination

## Homologous vaccination with A/H1N1 strain

P=0.07

P=0.00   P=0.00   P=0.00

| Parenteral | Plain 1 | Plain 2 | L9 | L9+MPL |
| 1.5 µg | 3 µg | 1.5 µg | 1.5 µg | 0.75 µg |

HI Titers (GMT): 800, 600, 400, 200, 0

## Homologous vaccination with A/H3N2 strain

P=0.13   P=0.09   P=0.13   P=0.52

| Parenteral | Plain 1 | Plain 2 | L9 | L9+MPL |
| 1.5 µg | 3 µg | 1.5 µg | 1.5 µg | 0.75 µg |

HI Titers (GMT): 800, 600, 400, 200, 0

## Homologous vaccination with B strain

P=0.91 —— P=0.91

P=0.00   P=0.01

| Parenteral | Plain 1 | Plain 2 | L9 | L9+MPL |
| 1.5 µg | 3 µg | 1.5 µg | 1.5 µg | 0.75 µg |

HI Titers (GMT): 4000, 3000, 2000, 1000, 0

# Fig. 2

Nasal Specific IgA responses recorded 14 days post-vaccination

### Homologous vaccination with A/H1N1 strain

| Parenteral - 1.5 µg | Plain 1 - 3 µg | Plain 2 - 1.5 µg | L9 1.5 µg | L9+MPL 0.75 µg |

### Homologous vaccination with A/H3N2 strain

| Parenteral - 1.5 µg | Plain 1 - 3 µg | Plain 2 - 1.5 µg | L9 1.5 µg | L9+MPL 0.75 µg |

### Homologous vaccination with B strain

| Parenteral - 1.5 µg | Plain 1 - 3 µg | Plain 2 - 1.5 µg | L9 1.5 µg | L9+MPL 0.75 µg |

# Fig. 3

Serum HI titers recorded 14 days post-heterologous vaccination

### Heterologous vaccination with A/H1N1 strain

### Heterologous vaccination with A/H3N2 strain

### Heterologous vaccination with B strain

Hypothesis = not significantly different from the parenteral group. p values > 0.05 mean the titer of the tested group is not statistically different from that of the parenteral group.

# Fig. 4

Nasal Specifiic anti-booster IgA responses recorded 14 days post-heterologous vaccination.

Heterologous vaccination with A/H1N1 strain

Heterologous vaccination with A/H3N2 strain

Heterologous vaccination with B strain

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9633738 A
- WO 2086232 A
- US 4912094 A
- GB 2220211 A
- US 5057540 A
- EP 0362279 B1
- WO 9910008 A
- WO 9602555 A
- WO 9113281 A
- EP 311863 B
- EP 516636 A
- DD 300833
- DD 211444
- DD 155875

### Non-patent literature cited in the description

- **J.M. WOOD et al.** An improved single radial immunodiffusion technique for the assay of influenza haemagglutinin antigen: adaptation for potency determination of inactivated whole virus and subunit vaccines. *J. Biol. Stand.,* 1977, vol. 5, 237-247
- **J. M. WOOD et al.** International collaborative study of single radial diffusion and immunoelectrophoresis techniques for the assay of haemagglutinin antigen of influenza virus. *J. Biol. Stand.,* 1981, vol. 9, 317-330
- **EYLES et al.** *BioDrugs,* 2000, vol. 13 (1), 35-59
- **FULK et al.** *J. Immunol.,* 1969, vol. 102, 1102-5
- **GLUCK et al.** *J. Virol.,* 1999, vol. 73, 7780-6
- **PETRESCU et al.** *Rev. Rom. Med-Virol.,* 1979, vol. 30, 109-115
- **OH et al.** *Vaccine,* 1992, vol. 10, 506-11
- **KUNO-SAKAI et al.** *Vaccine,* 1994, vol. 12, 1303-1310
- **MUSZKAT et al.** *Vaccine,* 2000, vol. 18, 1696-9
- **KIMURA et al.** *Acta Paediatr Jpn.,* 1988, vol. 30, 601-3
- **HASHIGUCCI et al.** *Vaccine,* 1996, vol. 14, 113-9
- Surfactant systems. Chapman and Hall, 1983
- Merck Index Entry 6858. Merck & Co. Inc, 1162
- Merck Index Entry 7742. Merck & Co. Inc, 1308
- Merck index. Merck & Co. Inc,
- **KENSIL, C. R.** *Crit Rev Ther Drug Carrier Syst,* 1996, vol. 12 (1-2), 1-55
- **KENSIL et al.** *J. Immunology,* 1991, vol. 146, 431-437
- **BOMMER, R.** *Pharmaceutical Technology Europe,* September 1999
- **LINA et al.** *Biologicals,* 2000, vol. 28, 95-103